# EUROPEAN PATENT SPECIFICATION

(11) **EP 0 349 242 B1**
(45) Date of publication and mention of the grant of the patent: **22.03.1995**
(21) Application number: 89306467.5
(22) Date of filing: 26.06.1989
(51) Int. Cl.: C07D 473/00, A61K 31/52

(54) **Therapeutic nucleosides**
Therapeutische Nuleoside
Nucléosides thérapeutiques

(30) Priority: 27.06.1988 GB 8815265
(43) Date of publication of application: 03.01.1990
(73) Proprietor: THE WELLCOME FOUNDATION LIMITED, London NW1 2BP (GB)
(72) Inventor: Daluge, Susan Mary, Chapel Hill North Carolina 27514 (US)
(74) Representative: Garrett, Michael

(56) References cited:
- CHEMICAL ABSTRACTS, vol. 108, no. 21, 23rd May 1988, page 749, abstract no.187147s, Columbus, Ohio, US; V.E. MARQUEZ et al.: "Synthesis of 2,'3'-dideoxycyclopentenyl carbocyclic nucleosides as potential drugs for the treatment of AIDS", & NUCLEOSIDES NUCLEOTIDES 1987, 6(1-2), 239-44
- CHEMICAL ABSTRACTS, vol. 110, no. 7, 13th February 1989, pages 15,16, abstract no.50717z, Columbus, Ohio, US; R. VINCENT et al.: "Potent and selective activity of a new carbocyclic nucleoside analog(carbovir: NSC 614846) against human immunodeficiency virus in vitro, BIOCHEM.BIOPHYS. RES. COMMUN. 1988, 156(2), 1046-53
- CHEMICAL ABSTRACTS, vol. 111, no. 5, 31st July 1989, page 650, abstract no.39839r, Columbus, Ohio, US; &JP-A-01 22 853 (ASAHI GLASS CO., LTD) 25-01-1989

## Description

The present invention relates to purine nucleoside analogues containing an unsaturated carbocyclic ring in place of the sugar residue, pharmaceutically acceptable derivatives thereof, and their use in therapy, particularly for the treatment or prophylaxis of certain viral infections.

AIDS is an immunosuppressive or immunodestructive disease that predisposes subjects to fatal opportunistic infections. Characteristically, AIDS is associated with a progressive depletion of T-cells, especially the helper-inducer subset bearing the OKT⁴ surface marker.

Human immunodeficiency virus (HIV) has been reproducibly isolated from patients with AIDS or with the symptoms that frequently precede AIDS. HIV is cytopathic and appears to preferentially infect and destroy T-cells bearing the OKT⁴ marker , and it is now generally recognized that HIV is the aetiological agent of AIDS.

Since the discovery that HIV is the aetiological agent of AIDS, numerous proposals have been made for anti-HIV chemotherapeutic agents that may be effective in treating AIDS sufferers. Thus, for example, European Patent Specification No. 196185 describes 3'-azido-3'-deoxythymidine (which has the approved name zidovudine), its pharmaceutically acceptable derivatives and their use in the treatment of human retrovirus infections including AIDS and associated clinical conditions. Vince et al., Antiviral Research, 9 (1/2), 120 (1988) describes certain carbocyclic purine nucleosides (in particular (±)-9-(cis-4-(hydroxymethyl)-2-cyclopentenyl) guanine and their use against HIV.

World wide, hepatitis B virus (HBV) is a viral pathogen of major consequence. It is most common in Asian countries, and prevalent in sub-Saharan Africa. The virus is aetiologically associated with primary hepatocellular carcinoma and is thought to cause 80% of the world's liver cancer. In the United States more than ten thousand people are hospitalised for HBV illness each year, an average of 250 die with fulminant disease.

The United States currently contains an estimated pool of 500,000-1-million infectious carriers. Chronic active hepatitis will develop in over 25% of carriers, and often progresses to cirrhosis. It is estimated that 5000 people die from HBV related cirrhosis each year in the USA, and that perhaps 1000 die from HBV-related liver cancer. Even when a universal HBV vaccine is in place, the need for effective anti-HBV compounds will continue. The large reservoir of persistently infected carriers, estimated at 220 million worldwide, will receive no benefit from vaccination and will continue at high risk for HBV induced liver disease. This carrier population serves as the source of infection of susceptible individuals perpetuating the instance of disease particularly in endemic areas or high risk groups such as IV drug abusers and homosexuals. Thus, there is a great need for effective antiviral agents, both to control the chronic infection and reduce progression to hepatocellular carcinoma.

Clinical effects of infection with HBV range from headache, fever, malaise, nausea, vomiting, anorexia and abdominal pains. Replication of the virus is usually controlled by the immune response, with a course of recovery lasting weeks or months in humans, but infection may be more severe leading to persistent chronic liver disease as outlined above. In "Viral Infections of Humans" (second edition, Ed., Evans, A.S. (1982) Plenum Publishing Corporation, New York), Chapter 12 describes in some detail, the aetiology of viral hepatitis infections.

Hepatitis B virus (HBV) is a small DNA containing virus which infects humans. It is a member of the class of closely related viruses known as the hepadnaviruses, each member of which selectively infects either mammalian or avian hosts, such as the woodchuck and the duck. Recent insights into the mechanism of replication of the hepadnavirus genome indicate the importance of reverse transcription of an RNA intermediate, suggesting that the reverse transcriptase is a logical chemotherapeutic target.

It has now been discovered that certain purine nucleoside analogues containing an unsaturated carbocyclic ring, as referred to below, are useful for the treatment or prophylaxis of viral infections for example hepatitis B and retroviral infections, especially AIDS.

According to a feature of the present invention, novel compounds of the formula (I) are provided: 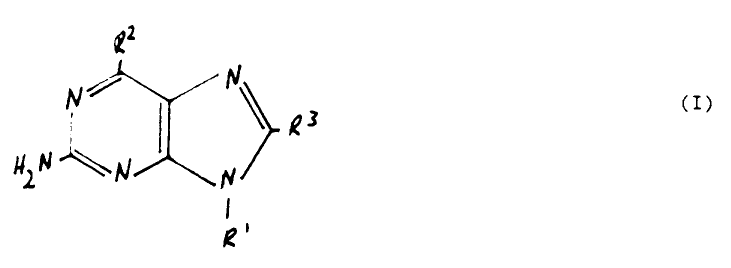
wherein R¹ represents 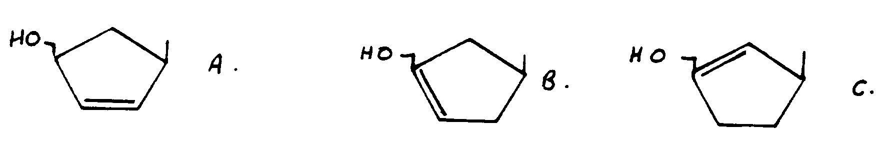
and R² represents branched or straight chain C₁₋₆ alkoxy (e.g. propyloxy or isopropoxy), optionally substituted by C₁₋₆ alkoxy, C₃₋₆ cycloalkyl (e.g. cyclopropylmethoxy); C₃₋₆ cycloalkyl; C₃₋₈ cycloalkyloxy (e.g. cyclobutyloxy or cyclopentyloxy); aryloxy (e.g. phenyloxy); aralky (e.g. benzyl) or aralkyloxy (e.g. benzyloxy) in which the aryl may optionally be substituted with C₁₋₄ alkyl, hydroxy or halogen; C₂₋₆ alkenylthio (e.g.allylthio); C₃₋₆ cycloalkylthio; C₁₋₆ alkylthio; arylthio or aralkylthio in which the aryl may optionally be substituted with C₁₋₄ alkyl, hydroxy, halogen or nitro; or R² represents a heterocyclic group containing an oxygen atom or one or two nitrogen atoms, and 3-7 carbon atoms with optional double bonds in the ring (e.g. piperidino, pyrrolidino or furfuryl) optionally containing one or more heteroatoms selected from sulphur and oxygen and optionally substituted on the ring by one or more C₁₋₄ alkyl, hydroxy or halogen groups, C₃₋₆ cycloalkylthio, aralkylthio in which the aryl may be substituted with C₁₋₄ alkyl, hydroxy or halogen; or R² represents an imidazolylthio group in which the imidazolyl moiety may be substituted with one or more substituents selected from C₁₋₄ alkyl and C-substituted with nitro; or R² represents an amino group which is mono- or di-substituted by one or two substituents selected from C₁₋₆ alkyl (e.g. methyl or ethyl), C₁₋₆ alkoxy (e.g. methoxy), C₁₋₆ hydroxyalkyl (e.g. hydroxyethyl), C₃₋₇ cycloalkyl (preferably C₃₋₆ cycloalkyl, e.g. cyclopropyl, cyclobutyl or cyclopentyl) optionally substituted by C₁₋₆ alkyl (e.g. cyclopropyl methyl), aryl (e.g. phenyl), aralkyl (e.g. benzyl) in which the aryl may optionally be substituted with C₁₋₄ alkyl, hydroxy or halogen, allyl optionally substituted with mono- or di-alkyl or alkoxy groups (e.g. dimethylallyl); and R³ represents hydrogen, amino or C₁₋₆ alkyl (e.g. methyl); or a pharmaceutically acceptable derivative thereof.

It should be noted that the invention also includes the individual optical and geometrical isomers of the compounds of formula (I) either alone or in admixture. Where reference is made to an alkyl moiety, this includes methyl, ethyl, propyl, butyl, petyl and hexyl.

According to a further feature of the present invention, we provide compounds of the formula: 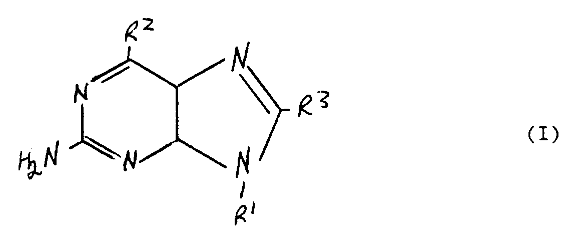
wherein R¹ represents a group of formula (A), (B) or (C) 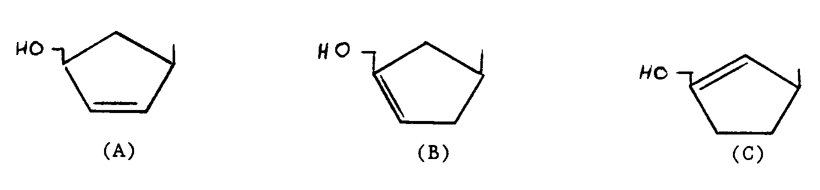
and R² represents C₁₋₆ alkoxy (e.g. propyloxy or isopropoxy), optionally substituted for example by C₃₋₆ cycloalkyl (e.g. cyclopropylmethoxy); C₃₋₈ cycloalkyloxy (e.g. cyclobutyloxy or cyclopentyloxy); aryloxy (e.g. phenyloxy), aralkyl (e.g. benzyl) or aralkyloxy (e.g. benzyloxy) in which the aryl may optionally be substituted with lower alkyl, hydroxy or halogen; C₃₋₆ cycloalkylthio; C₁₋₆ alkylthio; arylthio, or aralkylthio in which the aryl may optionally be substituted with lower alkyl, hydroxy, or halogen; or R² represents a heterocyclic group containing an oxygen atom or one or two nitrogen atoms, and 3-7 carbon atoms with optional double bonds in the ring (e.g. piperidino, pyrrolidino or furfuryl) optionally containing a sulphur and/or oxygen heteroatom and optionally substituted on the ring by one or more lower alkyl, hydroxy or halogen groups, C₃₋₆ cycloalkylthio, aralkylthio in which the aryl may be substituted with lower alkyl, hydroxy or halogen; or R² represents an imidazolylthio group in which the imidazolyl moiety may be substituted with lower alkyl and/or C substituted with nitro; or R² represents an amino group which is mono- or di-substituted by C₁₋₆ alkyl (e.g. methyl or ethyl), C₁₋₆ alkoxy (e.g. methoxy), hydroxy C₁₋₆ alkyl (e.g. hydroxyethyl) and/or C₃₋₆ cycloalkyl (e.g. cyclopropyl or cyclopentyl), aryl (e.g. phenyl), aralkyl (e.g. benzyl) in which the aryl may optionally be substituted with lower alkyl, hydroxy or halogen, allyl optionally substituted with mono- or di-alkyl or alkoxy groups (e.g. dimethylallyl); and R³ represents hydrogen or amino, and pharmaceutically acceptable derivatives thereof.

Preferred examples of compounds of formula (I) include those wherein R¹ represents A. Also preferred are compounds wherein R² is C₁₋₆ alkoxy for example methoxy or butoxy or R² is an amino group substituted by C₃₋₆ cycloalkyl (for example cyclopropyl or cyclobutyl) or R² is C₁₋₆ alkenyl or alkylthio (e.g. allylthio).

A preferred subgenus of formula (I) is as follows: 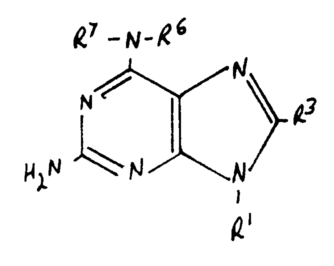
wherein R¹ represents 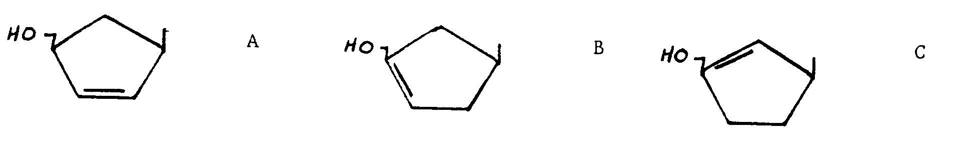
R³ represents hydrogen, amino or C₁₋₆ alkyl (e.g. methyl); R⁶ represents C₃₋₇ cycloalkyl (preferably C₃₋₆ cycloalkyl, e.g. cyclopropyl, cyclobutyl or cyclopentyl); and R⁷ represents a hydrogen atom or a substituent selected from C₁₋₆ alkyl (e.g. methyl or ethyl), C₁₋₆ alkoxy (e.g. methoxy), C₁₋₆ hydroxyalkyl (e.g. hydroxyethyl), C₃₋₇ cycloalkyl (preferably C₃₋₆ cycloalkyl, e.g. cyclopropyl, cyclobutyl or cyclopentyl) optionally substituted by C₁₋₆ alkyl (e.g. cyclopropyl methyl), aryl (e.g. phenyl), aralkyl (e.g. benzyl) in which the aryl may optionally be substituted with C₁₋₄ alkyl, hydroxy or halogen, allyl optionally substituted with mono- or di-alkyl or alkoxy groups (e.g. dimethylallyl); or a pharmaceutically acceptable derivative thereof.

Most preferably R¹ represents A, R³ represents hydrogen, R⁶ represents C₃₋₆ cycloalkyl and R⁷ represents a hydrogen atom.

The most preferred isomers are those in which the hydroxymethyl group is cis to the purine in compounds of formula (I) wherein R¹ is A.

Particularly preferred examples of compounds of formula (I) are:
a) (±)-cis-4-[2-amino-6-(cyclopropylamino)-9H-purin-9-yl]-2-cyclopentene-1-methanol;
b) (±)-cis-4-(2-amino-6-methoxy-9H-purin-9-yl)-2-cyclopentene-1-methanol;
c) (±)-cis-4-(2-Amino-6-ethoxy-9H-purin-9-yl)-2-cyclopentene-1-methanol;
d) (±)-cis-4-(2-Amino-6-isopropoxy-9H-purin-9-yl)-2-cyclopentene-1-methanol;
e) (±)-cis-4-(2-Amino-6( ethylthio)-9H-purin-9-yl)-2-cyclopentene-1-methanol;
f) (±)-cis-4-(6-(Allylthio)-2-amino-9H-purin-9-yl)-2-cyclopentene-1-methanol;
g) (±)-cis-4-(2-Amino-6-butoxy-9H-purin-9-yl)-2-cyclopentene-1-methanol;
h) (±)-cis-4-(2-Amino-6-cyclopentyloxy-9H-purin-9-yl)-2-cyclopentene-1-methanol;
i) (±)-cis-4-(6-(Allylamino)-2-amino-9H-purin-9-yl)-2-cyclopentene-1-methanol;
j) (±)-cis-4-(2-Amino-6-propoxy-9H-purin-9-yl)-2-cyclopentene-1-methanol;
k) (±)-cis-4-(2-Amino-6-(cyclopropylmethylamino-9H-purin-9-yl)-2-cyclopentene-1-methanol;
l) (±)-cis-4-(2-Amino-6-(cyclobutylamino)-9H-purin-9-yl)-2-cyclopentene-1-methanol;
m) (±)-cis-4-(2-Amino-6-(isobutylthio)-9H-purin-9-yl)-2-cyclopentene-1-methanol;

The compounds of formula (I) above and their pharmaceutically acceptable derivatives are hereinafter referred to as the compounds according to the invention.

In one aspect of the invention there are provided the compounds according to the invention for use in medical therapy particularly for the treatment or prophylaxis of retroviral infections and heptatis B viral infections.

Examples of retroviral infections which may be treated or prevented in accordance with the invention include human retroviral infections such as Human Immunodeficiency Virus (HIV), HIV-2 and Human T-cell Lymphotropic Virus (HLTV) e.g. HTLV-I or HTLV-IV infections. The compounds according to the invention are especially useful for the treatment or prophylaxis of AIDS and related clincial conditions such as AIDS-related complex (ARC), progressive generalised lymphadenopathy (PGL), AIDS-related neurological conditions, such as multiple sclerosis or tropical paraparesis, anti-HIV antibody-positive and HIV-positive conditions, Kaposi's sarcoma and thrombocytopenic purpura. The compounds may also be used in the treatment or prevention of psoriasis.

In a further aspect of the present invention there is included:-
a) Use of a compound according to the invention in the manufacture of a medicament for the treatment or prophylaxis of any of the above-mentioned infections or conditions.

By "a pharmaceutically acceptable derivative" is meant any pharmaceutically or pharmacologically acceptable salt, ester, salt of such ester, of a compound according to the invention or any other compound which, upon administration to the recipient, is capable of providing (directly or indirectly) a compound according to the invention, or an antivirally active metabolite or residue thereof.

Preferred esters of the compounds of the invention include carboxylic acid esters in which the non-carbonyl moiety of the ester grouping is selected from straight or branched chain alkyl e.g. n-propyl, t-butyl, n-butyl, alkoxyalkyl (e.g. methoxymethyl), aralkyl (e.g. benzyl), aryloxyalkyl (e.g. phenoxymethyl), aryl (e.g. phenyl optionally substituted by halogen, C₁₋₄ alkyl or C₁₋₄ alkoxy or amino); sulphonate esters such as alkyl- or aralkylsulphonyl (e.g. methanesulphonyl); amino acid esters (e.g. L-valyl or L-isoleucyl); and mono-, di- or tri-phosphate esters. The phosphate esters may be esterified by for example the following substituent CH₃(CH₂)ₙ wherein n is selected from 0-20.

With regard to the above-described esters, unless otherwise specified, any alkyl moiety present advantageously contains 1 to 18 carbon atoms, particularly 1 to 4 carbon atoms. Any aryl moiety present in such esters advantageously comprises a phenyl group.

Any reference to any of the above compounds also includes a reference to a pharmaceutically acceptable salt thereof.

Examples of pharmaceutically acceptable salts of the compounds according to the invention and pharmaceutically acceptable derivatives thereof include base salts, eg derived from an appropriate base, such as alkali metal (e.g. sodium), alkaline earth metal (e.g. magnesium) salts, ammonium and NW$\frac{\text{+}}{\text{4}}$ (wherein W is C₁₋₄ alkyl). Physiologically acceptable salts of a hydrogen atom or an amino group include salts of organic carboxylic acids such as acetic, lactic, tartaric, malic, isethionic, lactobionic and succinic acids; organic sulfonic acids such as methanesulfonic, ethanesulfonic, benzenesulfonic and p-toluenesulfonic acids and inorganic acids such as hydrochloric, sulfuric, phosphoric and sulfamic acids. Physiologically acceptable salts of a compound with a hydroxy group include the anion of said compound in combination with a suitable cation such as Na⁺, NH₄⁺, and NW₄⁺ (wherein W is a C₁₋₄ alkyl group).

The above compounds according to the invention and their pharmaceutically acceptable derivatives may be employed in combination with other therapeutic agents for the treatment or prophylaxis of the above infections or conditions. Examples of such further therapeutic agents include agents that are effective for the treatment or prophylaxis of viral infections or associated conditions such as 3'-azido-3'-deoxythymidine (zidovudine), 2',3'-dideoxynucleosides such as 2',3'-dideoxycytidine, 2',3'-dideoxy adenosine and 2',3'-dideoxyinosine, acyclic nucleosides (eg acyclovir), interferons such as α-interferon, renal excretion inhibitors such as probenicid, nucleoside transport inhibitors such as dipyridamole, as well as immunomodulators such as interleukin II and granulocyte macrophage colony stimulating factors. The component compounds of such combination therapy may be administered simultaneously, in either separate or combined formulations, or at different times, e.g. sequentially such that a combined effect is achieved.

The compounds according to the invention, also referred to herein as the active ingredient, may be administered for therapy by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient, the nature of the infection and the chosen active ingredient.

In general a suitable dose will be in the range of 3.0 to 120 mg per kilogram body weight of the recipient per day, preferably in the range of 6 to 90 mg per kilogram body weight per day and most preferably in the range 15 to 60 mg per kilogram body weight per day. The desired dose is preferably presented as two, three, four, five, six or more sub-doses administered at appropriate intervals throughout the day. These sub-doses may be administered in unit dosage forms, for example, containing 10 to 1500 mg, preferably 20 to 1000 mg, and most preferably 50 to 700 mg of active ingredient per unit dosage form.

Ideally, the active ingredient should be administered to achieve peak plasma concentrations of the active compound of from about 1 to about 75»M, preferably about 2 to 50»M, most preferably about 3 to about 30»M. This may be achieved, for example, by the intravenous injection of a 0.1 to 5% solution of the active ingredient, optionally in saline, or orally administered as a bolus containing about 1 to about 100 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

While it is possible for the active ingredient to be administered alone it is preferable to present it as a pharmaceutical formulation. The formulations of the present invention comprise at least one active ingredient, as defined above, together with one or more acceptable carriers thereof and optionally other therapeutic agents. Each carrier must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose) surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach. This is particularly advantageous for purine nucleoside derivatives as such compounds are susceptible to acid hydrolysis.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of an active ingredient.

The compounds according to the invention may also be presented for use in the form of veterinary formulations, which may be prepared, for example, by methods that are conventional in the art.

It should be understood that in addition to the ingredients particularly mentioned above the formulations of this invention may include other agents conventional in the art having regard to the type of formulation in question, for example, those suitable for oral administration may include such further agents as sweeteners, thickeners and flavouring agents.

The present invention further includes a process for the preparation of a compound according to the invention and pharmaceutically acceptable derivatives thereof which comprises either:
A) treating a compound of formula (II) 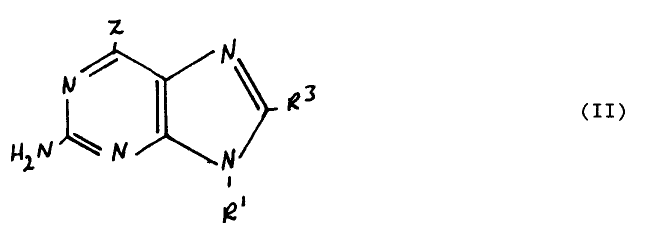 wherein R¹ and R³ are as hereinbefore defined and Z represents a precursor group for the said R² group with an agent or under conditions serving to convert the precursor Z group to the desired R² group; or Z represents a thio group onto which may be substituted an appropriate group to form a compound of formula (I) wherein R² is a substituted thio group or;
B) reacting a compound of formula 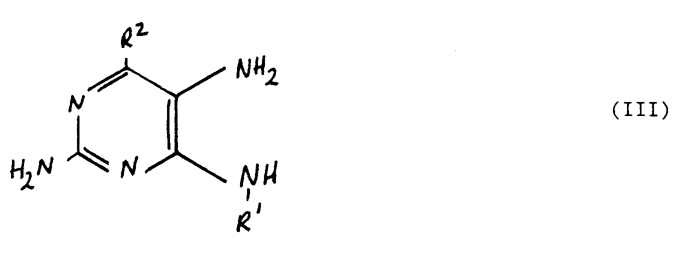 (wherein R¹ and R² are hereinbefore defined) or a pharmaceutically acceptable derivative thereof, with an agent serving to effect formation of the imidazole ring in the desired compound of formula (I); or
   i) where a compound of formula (I) is formed, converting said compound to a pharmaceutically acceptable derivative thereof; or
   ii) where a pharmaceutically acceptable derivative of a compound of formula (I) is formed, converting the said derivative to the parent compound of formula (I) or to a further such derivative.

Process A) above may be carried out in conventional manner, for example, by treatment of a compound of formula (II) in which Z represents a leaving group, e.g. a halo such as a chloro group, with, for example, an alkali metal (e.g. sodium) or alkali metal hydride (e.g. sodium hydride) and an appropriate alcohol at reflux or a temperature greater than 50°C preferably in an organic solvent.

Alternatively the compound of formula (II) may be treated with an appropriate amine or amino hydrochloride to introduce a substituted amino group as defined above at reflux or at a temperature greater than 50°C preferably in the presence of an organic solvent, for example methanol, ethanol. Alternatively, a compound of formula (II) wherein Z is a thio group may be treated with an appropriate halide under nitrogen.

Process B) may be carried out, e.g., by reacting a compound of formula (III) with formic acid or a reactive formic acid derivative, triethylorthoformate or diethoxymethyl acetate, in a solvent such as a dimethylacetamide or acetonitrile at an elevated temperature, preferably at 75-90°C. This reaction is conveniently effected by the addition of slightly more than one equivalent of a strong anhydrous acid eg. with 1.1 equivalents of ethanesulfonic acid per equivalent of compound of formula (III), in which case lower temperatures, 25°C., are used.

In process A) the starting material of formula (II) may be prepared, for example, by firstly cyclising a compound of formula (III) above in an analogous manner to that described for process B) above.

Other reagents may be useful for cyclisation of compounds of formula (II) to give compounds of formula (I) where R³ is not hydrogen. For example triethyl or trimethylorthoacetate with acetic anhydride at 70-120°C for several hours gives R³=CH₃ (see H.C. Koppel and R.K. Robins, J.Org.Chem 1958, 1457), R³=NH₂ may be obtained by cyclisation with ethoxycarbonyl isothiocyanate (see R. Esmail and F.Kurzer, Synthesis 1975, 301; L.B. Towsend, et al., J.Heterocyclic Chem. 1984, 21, 1245). This initially gives R³=NHCO₂ Et which is then hydrolysed in base (e.g. aqueous sodium hydroxide) to R³=NH₂. Cyclisation with potassium ethylxanthate (W.T. Stolle, J.C Sih, R.S.P. Hsi, J. Label. Compound Radiopharm. 1988, 891) in ethanol at 80°C gives R³=SH. Alkylation of the SH with alkyl halides and base (e.g. potassium carbonate in DMF gives R³=SMe, SEt.

A compound of formula (I) may be converted into a pharmaceutically acceptable ester by reaction with an appropriate esterifying agent, e.g. an acid halide or anhydride. The compound of formula (I), including esters thereof, may be converted into pharmaceutically acceptable salts thereof in conventional manner, e.g. by treatment with an appropriate base. An ester or salt of a compound of formula (I) may be converted into the parent compound, e.g. by hydrolysis.

The optical and geometric isomers of the compounds of formula (I) may be resolved or isolated in conventional manner, e.g. by chromatographic separation of diastereomeric esters prepared by acylation of the hydroxyl on the cyclopentenyl moiety with appropriate optically active carboxylic acid derivatives as, e.g., with naproxen (J.Org.Chem., 51, 1287 (1986)). The cyclopentenyl precursors of the compounds of formula (III ), may also be resolved by fractional crystallization of salts formed with optically active carboxylic acids (e.g. L-tartaric acid and its derivatives). Alternatively, enzymatic resolution may be achieved as in J.Med.Chem., 30, 746 (1987) and J.Med.Chem. 28, 1385 (1985).

The following Examples are intended for illustration only and are not intended to limit the scope of the invention in any way. The term 'active ingredient' as used in the Examples means a compound of formula (I) or a pharmaceutically acceptable derivative thereof.

### Example 1

### (±)-cis-4-[(2-Amino-4-chloro-6-pyrimidinyl)amino]-2-cyclopentene-1-methanol

cis-4-Acetamidocyclopent-2-enemethyl acetate [S.Daluge and R.Vince, J.Org.Chem. 1978, 43, 2311] (14.88g, 0.073mol) and barium hydroxide octahydrate (46.19g, 0.146 mol) were refluxed in water (300mL) under nitrogen for 18 hours. The resulting solution was neutralized with carbon dioxide. The precipitate was washed with water, then ethanol. The combined filtrate-wash was evaporated to a syrup (11.16g) which was condensed with 2-amino-4,6-dichloropyrimidine (23.91g, 0.146mol) and triethylamine (30.5mL, 0.219mol) in refluxing 1-butanol (100mL) for 1.5 hours. After addition of 1N NaOH (73mL), the resulting mixture was evaporated to dryness and the residual solid slurried in CHCl₃ (200mL). Unreacted 2-amino-4,6-dichloropyrimidine was filtered off and washed with chloroform (100mL). The chloroform filtrate-wash was concentrated and chromatographed on a silica gel column. Additional pyrimidine starting material was eluted with 2.5% methanol-chloroform. The title compound was eluted with 3.5% methanol-chloroform as an off-white solid foam (15.90g, 91%); 'H-NMR (Me₂SO-d₆) 1.15-1.28 and 2.26-2.41 (2m,2,CH₂), 2.60-2.71 (m,1,1'-H), 3.4 (m overlapping H₂O, CH₂OH), 4.625 (t,J=5.3,1,CH₂OH), 4.95 (br s,1,CH-N), 5.67-5.87 (m,2,CH=CH), 6.38 (br s,1,NH₂), 7.12(br s,1,NH); MS (CP)M+1, 241,243.

| | | | | |
|---|---|---|---|---|
| Anal. calc. for C₁₀H₁₃N₄OC1.0.2H₂O : | C,48.99; | H,5.55; | N,22.85; | Cl,14.46. |
| Found : | C,49.10; | H,5.57; | N,22.81; | Cl,14.40. |

### Example 2

### (±)-cis-4-[[2-Amino-6-chloro-5-[(4-chlorophenyl)azo]-4-pyrimidinyl]amino]-2-cyclopentene-1-methanol

(±)-cis-4-[(2-Amino-4-chloro-6-pyrimidinyl)amino]-2-cyclopentene-1-methanol from Example 1 (11.58g, 48.1mmol) and sodium acetate trihydrate (97g) were dissolved in glacial acetic acid (225mL) and water (225mL). A cold solution (0.5°C) of 4-chlorobenzenediazonium chloride was prepared from 4-chloroaniline (6.74g, 52.8mol), concentrated hydrochloric acid (14.7mL) water (52mL), and sodium nitrite (4.01g, 58.2mMol in 47mL of water). This cold solution was added dropwise over 5 minutes to the first solution. The resulting yellow precipitate was filtered after 18 hours, washed with water, and extracted with ethanol to give title compound as dark yellow powder (12.56g, 69%), Mp 218-220°C dec; 'H-NMR (Me₂SO-d₆) 10.25(d,1,NH), 7.69 and 7.54 (both, J=8.9,C₆H₄) overlapping 7.6 (br,6,NH₂), 5.80-5.95 (m,2,CH=CH) 5.24(m,1,CHN), 4.75(t,1,CH₂OH), 3.41(t,1,CH₂OH), 3.41(t,2,CH₂OH), 2.75(m,1,CH), 2.41(m,1,CH), 1.44-1.53(m,1,CH).

| | | | | |
|---|---|---|---|---|
| Anal calc. for C₁₆H₁₆N₆Cl₂O : | C,50.67; | H,4.25; | N,22.16; | Cl,18.70. |
| Found : | C,50.59; | H,4.29; | N,22.10; | Cl,18.66 |

### Example 3

### (±)-cis-4-[(2,5-Diamino-4-chloro-6-pyrimidinyl)-amino]-2-cyclopentene-1-methanol.

The title compound of Example 2 (11.67g) was suspended in ethanol (235mL), glacial acetic acid (30mL), and water 235mL). The mixture was heated to reflux under nitrogen. Zinc dust (13.5g) was added in small portions over 30 minutes during which time the compound dissolved. The reaction was heated an additional 20 minutes, and then the excess zinc was filtered of from the hot solution, and it was washed with ethanol. The filtrates were evaporated, and the residue was purified on a silica gel column eluting with chloroform (1L) and chloroform:methanol/4:1 (1.8L). The fractions containing the product were combined, and the solvent was removed under reduced pressure to give the title compound as a red-orange oil (11.2g, >100% yield). A pure sample was obtained during another small scale reaction to obtain the product as a light yellow solid in a 76% yield; ¹H-NMR (Me₂SO-d₆) 1.29 and 2.39 (m, 2, CH₂), 2.69 (t, 1, 1'-H), 3.37 (d, 2, CH₂OH), 3.91 (br, 2, NH₂), 4.60 (br, 1, CH₂OH), 5.02 (m, 1, CHNH), 5.56 (br s, 2, NH₂), 5.74 (m, 1, = CH), 5.86 (m, 1, = CH), 6.36 (d, 1, CHNH).

### Example 4

### (±)-cis-4-(2-Amino-6-chloro-9H-purin-9-yl)-2-cyclopentene-1-methanol

The title compound of Example 3 (about 9.7g) was dissolved in diethoxymethyl acetate (100g), and refluxed for two days. The solvent was removed under high vacuum at 50°C, and dioxane (40mL) and 0.5N HCl (60mL) was added. The reaction was stirred at room temperature for 1.25 hours, and then chilled. The reaction was neutralized to pH7 with cold 5N sodium hydroxide, and then it was extracted with chloroform : methanol/3:1 several times. The organic layers were dried with magnesium sulphate, filtered, and evaporated. The residue was purified by chromatography on a silica gel column, eluting with CHCl₃:2% MeOH to give 3.7g (46% yield) of the title compound, Mp 138-139°C. ¹H-NMR (Me₂SO-d₆) 1.63 and 2.61 (m, 2, CH₂), 2.87 (m, 1, 1'-H), 3.44 (d, 2, CH₂OH), 5.44 (m, 1, CH-N), 5.89 (m, 1, = CH), 6.14 (m, 1, = CH), 6.82 (br s, 2, NH₂), 8.02 (s, 1, 8-H). (CH₂OH not seen - under H₂O peak) uv : pH 1 lambda max 315 ( 7370), 218 (26200); lambda sh 239.5 (5650). pH 7.4 lambda max 307 ( 8000), 245.5 (4600), 223 (26400). MS (E1) 265,267 (m) (Cl) 266,268 (m + 1).

| | | | | |
|---|---|---|---|---|
| Anal. calc. for C₁₁H₁₂N₅Cl0.2H₂O : | C,43.79; | H,5.35; | N,23.21; | Cl,11,75. |
| Found : | C,43.67; | H,5.29; | N,23.05; | Cl, 11.70 |

### Example 5

### (±)-cis-4-[2-Amino-6-(cyclopropylamino)-9H-purin-9-yl]-2-cyclopentene-1-methanol

The title compound of Example 4 (0.50g) was dissolved in ethanol (40mL), and cyclopropylamine (0.65mL, 5 equivalent) was added. The reaction was refluxed under nitrogen for 6 hours. An additional 0.65mL of cyclopropylamine was added, and the reaction refluxed for an additional 5.5 hours. The solvents were evaporated, and chloroform (25mL) and saturated sodium bicarbonate solution (5mL) was added. The aqueous layer was extracted several times with CHCl₃ to obtain the crude product. This was purified on a silica gel column eluting with ethyl acetate : 3% methanol to give 0.43g (80%) of the title compound. This was recrystallised from acetonitrile to give 0.30 g of product; Mp 70°C shrivels, 85°C gels; ¹H-NMR (Me₂SO-d₆) 0.56 and 0.63 (2m, 4, CH₂CH₂), 1.56 and 2.60 (2m, 2, 5'-CH₂), 2.85 (m, 1, 1'-H), 3.02 (m, 1, CH-NH), 3.43 (t, 2, CH₂OH), 4.71 (t, 1, CH₂OH), 4.28 (m, 1, 4'-H), 5.77 (s, 2, NH₂), 5,84 (m, 1, = CH₂), 6.09 (m, 1, =CH), 7.23 (d, 1, NH-CH), 7.58 (s, 1, purine-8-H); ms (CI) 287 (m+); uv pH 1: lambda max 296 ( 12850), 255 (9800), 210.5 (18100); lambda sh 221 (16300). pH 7.4 : max 284.5 (14300), 259.5 (8250), 216 (21100).

| | | | |
|---|---|---|---|
| Anal. calc. for C₁₄H₁₈N₆O.0.25 H₂O : | C, 57.82; | H,6.41; | N,28.90. |
| Found ; | C,57.84; | H,6.45; | N,28.86 |

### Example 6

### (±)-cis-4-(2-Amino-6-methoxy-9H-purin-9-yl)-2-cyclopentene-1-methanol

(±)-cis-4-(2-Amino-4-chloro-9H-purin-9-yl)-2-cyclopentene-1-methanol from Example 4 (0.53g, 2.0mmol) was dissolved in methanol (25mL) and a solution of sodium (0.23g, 10 m.equiv.) and methanol (20mL) added. After 1.0 hour of reflux, the solution was cooled, neutralized with hydrochloric acid. Evaporated solvent and chromatographed residue on silica gel. Product was eluted as a white solid foam (0.44g) with 5% methanol-ethylacetate; 'H-NMR (Me₂SO-d₆) 1.57 and 2.60 (both m,2,CH₂), 2.85 (m,1,1'-H), 3.43 (t, 2, CH₂OH), 3.94 (s, 3, OCH₃), 4.70 (t, 1, CH₂OH, 5.42 (m,1,CH-N), 5.86 and 6.11 (m,2,CH=CH), 6.37 (s,2,NH₂), 7.75 (s,1,purine-8H).

| | | | |
|---|---|---|---|
| Anal. calc. for C₁₂H₁₅N₅O₂.0.05 EtOAc.0.2H₂O.0.3MeOH: | C,53.83; | H,6.14; | N,25.11. |
| Found : | C,53.87; | H,6.09; | N,25.07. |

### Example 7

### (±)-cis-4-(2-Amino-6-ethoxy-9H-purin-9-yl)-2-cyclopentene-1-methanol

A flask was charged with ethanol (33ml) and sodium (0.172g, 7.5mmol). After all of the sodium had dissolved (±)-cis-4-(2-amino-6-chloro-9H-purin-9-yl)-2-cyclopentene-1-methanol(0.40g,1.5mmol) from Example 4 was added and the solution was brought to reflux for 0.5 hours. The solution was allowed to cool to room temperature before neutralization with 1.0N HCl. The solution was then concentrated and the residue partitioned between chloroform and water. The organic layer was dried with MgSO₄, filtered and concentrated. The residues were then placed on a silica gel column and eluted with 2% methanol in chloroform to yield a yellow glass (0.28g, 67.8%), 'H-NMR(Me₂SO-d₆) δ7.74 (s,1H, purine H-8), 6.35(br s, 2H, NH₂), 6.15 and 6.07 and 5.91-5.82(both m, 2H,CH=CH) 5.49-5.35(br m,1H,CHN) 4.71(t,J=5.3Hz,1H,OH), 4.43(a,J=7.0Hz,OCH₂ CH₃), 3.50-3.39(m,2H, OCH₂), 2.95-2.78(br m,1H,H-1'), 2.70-2.52(br m, overlapping solvent, 0.5 CH₂), 1.68-1.52(br m,1H,0.5 CH₂), 1.33(+,J=7.1Hz,3H,CH₃).

| | | | |
|---|---|---|---|
| Anal. calc. for C₁₃H₁₇N₅O₂ : | C,56.72; | H,6.22; | N,25,44. |
| Found: | C,56.48; | H,6.28; | N,25.20. |

### Example 8

### (±)-cis-4-(2-Amino-6-isopropoxy-9H-purin-9-yl)-2-cyclopentene-1-methanol

A flask was charged with sodium hydride (60% oil dispersion, 240mg, ∼6mmol) which was washed with hexanes before the addition of iso-propanol (20mL) and (±)-cis-4-(2-amino-6-chloro-9H-purin-9-yl)-2-cyclopentene-1-methanol (0.318g, 1.19mMol) from Example 4. This solution was heated at 75°C for 2 hours before being allowed to cool to room temperature and neutralized by addition of 1.0N HCl. The solution was concentrated and the residue was placed on a silica gel column which was eluted with 10% methanol-chloroform. Crystallisation from ethanol: water-1:1 yielded off-white crystals (265mg,77%) Mp 188-191°C,'H-NMR (Me₂SO-d6) δ7.72(s,1H,purine H-8), 6.28(s,2H,NH₂), 6.09 and 5.87 (both m, 2H,CH=CH), 5.50-5.37(m,2H,CHN,CHO), 4.69(t,J=5.3,1H,OH), 3.43(m,2H,CH₂OH) 2.86-2.81 (br m,1H,CH), 2.67-2.51 and 1.64-1.52(2m,2H,CH₂) 1.31(d,J=6.2,(CH₃)₂CH).

| | | | |
|---|---|---|---|
| Anal. calc. for C₁₄H₁₉N₅O₂; | C,58.12; | H,6.62; | N,24.20. |
| Found: | C,58.20; | H,6.66: | N,24.20. |

### Examples 9 and 10

### (±)-cis-2-Amino-1,9-dihydro-9-[(4-hydroxymethyl)-2-cyclopenten-1-yl]-6H-purin-6-thione and (±)-cis-4-(2-Amino-6-(propylthio)-9H-purin-9-yl)-2-cyclopentene-1-methanol

(±)-cis-4-(2-Amino-6-chloro-9H-purin-9-yl)-2-cyclopenten-1-methanol (4.18g,15.7mmol) from Example 4 and thiourea (1.32g,17.3mmol) were refluxed in n-propanol for 17 hours. The resulting mixture was filtered and the solid washed with n-propanol to give (±)-cis-2-amino-1,9-dihydro-9-[(4-hydroxymethyl)-2-cyclopenten-1-yl)-6H-purin-6-thione as yellow powder (2.19g,53%); Mp 235-238°C, 'H-NMR(DMSO-d₆) δ; 11.85(5,1,NH); 7.77(s,1,H-8), 6.76 (br s, 2 NH₂), 6.16 and 5.86 (both m, 2, CH=CH), 5.35(m,1,CH-N), 4.70(m,1,OH), 3.42 (br m, overlapping H₂O, CH₂-O), 2.85 (br m,1,CH), 2.30 (m, overlapping solvent, 0.5 CH₂), 1.60 (m,1, 0.5 CH₂).

| | | | | |
|---|---|---|---|---|
| Anal. calc. for C₁₁H₁₃N₅OS: | C,50.18; | H,4.98; | N,26.60; | S,12.18. |
| Found: | C,50.10; | H,5.00; | N,26.50; | S,12.10. |

Chromatography on silica gel of the propanol filtrate contents gave a second product on elution with 5% MeOH-EtOAc. Crystallisation from acetonitrile gave (±)-cis-4-(2-amino-6-(propylthio)-9H-purin-9-yl)-2-cyclopenten-1-methanol as a yellow powder (0.441g, 10%), Mp 128-130°C; 'H-NMR (DMSO-d₆) δ: 7.84(s,1,H-8), 6.46(br s, 2, NH₂), 6.15 and 5.90 (both m, 2, CH=CH), 5.45 (br m, 1,CH-N), 4.73(t, J=5.4,1,OH), 3.45(m,2,CH₂-O), 3.25(t,J=7.3,2,s-CH₂), 2.90(br m,1,CH), 2.70-2.55(m,1, 0.5 CH₂), 1.80-1.50(m,3, 0.5 CH₂ overlapping CH₂ of propyl), 1.0 (t, J-7.3,3,CH₃).

| | | | | |
|---|---|---|---|---|
| Anal. calc. for C₁₄H₁₉N₅OS: | C,55.06; | H,6.27; | N,22.93; | S,10.50. |
| Found: | C,55.15; | H,6.25; | N,22.91; | S,10.58. |

### Example 11

### (±)-cis-4-(2-Amino-6-(methylthio)-9H-purin-9-yl)-2-cyclopentene-1-methanol

A solution of (±)-cis-2-amino-1,9-dihydro-9-[(4-hydroxymethyl)-2-cyclopentenl-yl)]-6H-purin-6-thione (0.50g, 1.89mmol) from Example in 1N NaOH (1.89mL) was stirred under N₂ with methyliodide (0.54g, 3.79mmol) for 30 minutes. The mixture was extracted with CHCl₃ (3 x 100ml). Extracts were dried (MgSO₄), solvent evaporated and the residual solid chromatographed. Product was eluted from a silica gel column with 10% MeOH-CHCl₃. Crystallisation of such a sample from acetonitrile gave title compound as yellow crystals (0.410g, 78%); Mp. 152-154°C; 'H-NMR(DMSO-d₆) δ; 7.84((s,1,H-8), 6.49(br s, 2, NH₂), 6.15 and 5.90 (both m, 2, CH=CH), 5.45 (br m, 1,CH-N), 4.73 (t, J=5.3, 1, OH), 3.45(m,2,CH₂-O), 2.90 (br m, 1, CH), 2.70-2.55 (m, overlapping S at 2.57, 4, 0.5 CH₂ and CH₃), 1.65-1.55 (m, 1, 0.5 CH₂).

| | | | | |
|---|---|---|---|---|
| Anal. calc. for C₁₂ H₁₅ N₅ OS: | C,51.97; | H,5.45; | N,25.25; | S,11.56 |
| Found: | C,51.98; | H,5.48; | N,25.21; | S,11.65 |

### Example 12

### (±)-cis-4-(2-Amino-6-((4-nitrobenzyl)thio)-9H-purin-9-yl)-2-cyclopentene-1-methanol

A solution of (±)-cis-2-amino-1,9-dihydro-9-[(4-hydroxymethyl-2-cyclopenten-1-yl)-6H-purin-6-thione (0.50g, 1.89mmol)from Example 10 in DMF(5mL) was stirred with K₂CO₃ (0.26g, 1.89mmol) and p-nitrobenzylbromide (0.41g, 1.89mmol) under nitrogen for 12 hours. The mixture was partitioned between H₂O (5mL) and CHCl₃ (3x50mL). The CHCl₃ extracts were dried (MgSO₄), concentrated to a yellow oil, and the oil chromatographed. Elution of a silica gel column with 10% MeOH-CHCl₃ gave title compound as a yellow powder (0.545g,73%), Mp. 199-201°C; H-NMR(DMSO-d₆) δ; 8.15 (AB,J=8.8H₂,2, 0.5 C₆H₄), 7.76 (AB,J=9.0, 2, 0.5 C₆H₄), 7.83 (s, 1, H-8), 6.62 (s, 2, NH₂), 6.10 and 5.85 (both m, 2, CH=CH), 5.40 (br m, 1, CH-N), 4.70 (t,J=5.3,1, OH), 4.63 (s, 2, CH₂-S), 3.42 (m, 2, CH₂-O), 2.85 (br m, 1, CH), 2.70-2.50 (m, overlapping solvent, 0.5 CH₂), 1.70-1.50 (m, 1, 0.5 CH₂).

| | | | | |
|---|---|---|---|---|
| Anal. calc. for C₁₈H₁₈N₆O₃S: | C,54.26; | H,4.55; | N,21.09; | S,8.05 |
| Found: | C,54.17; | H,4.56; | N,21.05; | S,8.11 |

### Example 13

### (±)-cis-4-(2-Amino-6-((1-methyl-4-nitro-1H-imidazol-5-yl)thio)-9H-purin-9-yl)-2-cyclopentene-1-methanol

To a solution of (±)-cis-2-amino-1,9-dihydro-9-[(4-hydroxymethyl)-2-cyclopenten-1-yl)-6H-purin-6-thione (0.50g, 1.89mmol) from Example 10 in 1N NaOH (1.89mL) was added 1-methyl-4-nitro-5-chloro-imidazole (0.31g, 1.89mmol). The solution was stirred under nitrogen overnight and the resulting precipitate filtered after addition of H₂O (3mL). Chromatography of the precipitate on silica gel gave title compound, eluted with 10% MeOH-CHCl₃ as yellow powder (0.638, 87%), Mp. 207-208°C; 'H-NMR(DMSO-d₆) δ; 8.19(s,1 imidazolyl CH), 7.89(S, 1, H-8), 6.55 (br, s, 2, NH₂), 6.15 and 5.85 (both m, 2, CH=CH), 5.40 (br m, 1, CH-N), 4.70 (t,J=5.3Hz, 1, OH), 3.65 (s, 3, CH₃), 3.40 (m, 2, OCH₂), 2.85 (br m, 1, CH), 2.70-2.50 (m, overlapping solvent, 0.5 CH₂), 1.70-1.50 (m, 1, 0.5 CH₂).

| | | | | |
|---|---|---|---|---|
| Anal. calc. for C₁₅H₁₆NO₃S-1H₂O: | C,44.33; | H,4.46; | N,27.57; | S,7.89 |
| Found: | C,44.22,44.12; | H,4.46,4.49; | N,27.52,27.46; | S,7.81 |

### Example 14

### (±)-cis-4-(2-Amino-6-(ethylthio)-9H-purin-9-yl)-2-cyclopentene-1-methanol

To a solution of (±)-cis-2-amino-1,9-dihydro-9-[(4-hydroxymethyl)-2-cyclopenten-1-yl)-6H-purin-6-thione (0.50g, 1.89mmol) in 1N NaOH (1.89mL) was added ethyliodide (0.29g, 1.89mmol) and dioxane (1 mL). The solution was stirred at 25°C under nitrogen for 1.25 hours. The resulting mixture was extracted with CHCl₃ (3x50mL), dried (MgSO₄) and evaporated to give crude product as yellow oil. Chromatography on silica gel gave title compound, eluted with 10% MeOH-CHCl₃ and solidfied to yellow powder in acetonitrile (0.445g, 80%); Mp.123-125°C; 'H-NMR(DMSO-d₆) δ;7.82 (s, 1, H-8), 6.43 (br, s, 2, NH₂), 6.15 and 5.85 (both m, 2, CH=CH), 5.43 (m, 1, CH-N), 4.70 (t,J=5.4, 1, OH), 3.43 (m, 2, CH₂-O), 3.23 (q,J=7.3, overlapping H₂O, S-CH₂), 2.85 (br m, 1, CH), 2.70-2.55 (m, 1, 0.5 CH₂), 1.70-1.50 (m, 1, 0.5 CH₂), 1.30 (t,J=7.3, 3, CH₃).

| | | | | |
|---|---|---|---|---|
| Anal. calc. for C₁₃H₁₇N₅OS: | C,53.51; | H,5.83; | N,24.19; | S,10.99. |
| Found: | C,53.32; | H,5.93; | N,24.00; | S,10.94 |

### Example 15

### (±)-cis-4-(2-Amino-6-(allylthio)-9H-purin-9-yl)-2-cyclopentene-1-methanol

To a solution of (±)-cis-2-amino-1,9-dihydro-9-[(4-hydroxymethyl)-2-cyclopenten-1-yl]-6H-purine-6-thione (0.50g, 1.89mmol) from Example 10 in 1N NaOH (1.89mL), was added allylbromide (0.229g, 1.89mmol) and dioxane (1mL). The solution was stirred at 25°C under nitrogen for 1 hour. The mixture was extracted with CHCl₃ (3x50) and the CHCl₃ extracts dried (MgSO₄) and concentrated to a yellow oil. Chromatography on silica gel gave the title compound, which was eluted with 10% MeOH-CHCl₃ and solidified to yellow powder in acetonitrile, (0.436g, 76%), Mp.108-110°C; H-NMR (DMSO-d₆) δ; 7.83(S, 1, H-8), 6.49 (br s, 2, NH₂), 6.15 and 5.85 (both m, overlapping 6.0, m, total 3, CH=CH, and CH=CH₂). 5.45 (m, overlapping dd centered at 5.35, 2, CH-N, and 0.5 =CH₂), 5.10 (dd, 1, 0.5 =CH₂), 4.70 (t,J=5.3Hz, 1, OH), 3.95 (d,J=6.8Hz, 2, S-CH₂), 3.45 (m, 2, CH₂-O), 2.85 (br m, 1, CH), 2.60 (m, 1, 0.5 CH₂), 1.60 (m, 1, 0.5 CH₂).

| | | | | |
|---|---|---|---|---|
| Anal. calc. for C₁₄H₁₂N₅OS: | C,55.42; | H,5.65; | N,23.08; | S,10.57 |
| Found: | C,55.37; | H,5.70; | N,23.03; | S,10.47 |

### Example 16

### (±)-cis-4-(2-Amino-6-butoxy-9H-purin-9-yl)-2-cyclopentene-1-methanol

A flask was charged with sodium hydride (60% oil dispersion 300mg, 7.5mmol) which was then washed with hexanes before the addition of butanol (10mL) containing (±)-cis-4-(2-amino-6-chloro-9H-purin-9-yl)-2-cyclopentene-1-methanol (800mg, 3mmol) from Example 4. The solution was stirred at reflux for 2 hours and then neutralized by the addition of 1.0 N NaOH. Concentration of the solution afforded the crude product which was purified by elution from a silica gel column with 5% methanol - chloroform. (810mg, 94%). Crystallisation of such a sample from ethanol/acetonitrile gave an off-white powder, Mp. 122-123°C, 'H-NMR (Me₂SO-d-₆) δ 7.73 (S, 1H, purine H8), 6.12 and 5.86 (m,2H,CH=CH), 5.41 (br m, 1H, NCH) 4.71 (unressolved t, 1H, OH), 4.38 (t,J=6.6Hz, 2H, OCH₂ butyl), 3.42 (m, 2H, OCH₂), 2.85 (br m, 1H, CH), 2.60 (m, 1H, 0.5CH₂ cyclopentene), 1.75-1.35 (m, 5H, 0.5CH₂ cyclopentene, CH₂ CH₂) 0.92 (t,J=7.3Hz, 3H, CH₃).

| | | | |
|---|---|---|---|
| Anal. calc. for C₁₅H₂₁N₅O₂: | C,59.39; | H,6.98; | N,23.09. |
| Found: | C,59.56; | H,7.07; | N,22.87. |

### Example 17

### (±)-cis-4-(2-Amino-6-cyclopentyloxy-9H-purin-9-yl)-2-cyclopentene-1-methanol

A flask was charged with sodium hydride (60% oil dispersion, 185mg, 4.6mmol) which was then washed with hexanes before the addition of cyclopentanol (10mL). The resulting mixture was heated and (±)-cis-4-(2-Amino-6-chloro-9H-purin-9-yl)-2-cyclopentene-1-methanol (0.53g, 2mmol) from Example 4 was added. After stirring at 100°C for 0.75 hours the solution was neutralized by the addition of 1.0N HCl. Concentration of the solution afforded the crude product which was purified by elution from a silica gel column with 5% methanol-chloroform (0.30g 47.6%). Crystallisation of such a sample from ethanol-acetonitrile gave an off white powder, Mp.188-190°C; 'H-NMR (Me₂SO-d₆) δ 7.74 (S, 1H, purine H8), 6.33 (br s, 2H NH₂), 6.12 and 5.87 (m, 2H, CH=CH), 5.60 (m, 1H, OCH), 5.42 (m, 1H, NCH), 4.73 (t,J=5.1Hz, 1H, OH), 3.44 (m, 2H, OCH₂) 2.87 (br m, 1H, CH), 2.63-2.57 (m,1H, 0.5 CH₂ cyclopentane), 1.98 (br m, 2H, cyclopentane), 1.80-1.57 (br m, 7H, 0.5 CH₂ cyclopentene, 3CH₂).

| | | | |
|---|---|---|---|
| Anal. calc. for C₁₆H₂₁N₅O₂: | C,60.94; | H,6.71; | N,22.21. |
| Found: | C,60.99; | H,6.73; | N,22.20. |

### Example 18

### (±)-cis-4-(2-Amino-6-cyclopentylamino-9H-purin-9-yl)-2-cyclopentene-1 -methanol

A solution of (±)-cis-4-(-2-Amino-6-chloro-9H-purin-9-yl)-2-cyclopentene-1-methanol (0.53g, 2mmol) from Example 4, triethylamine (2.00g, 20mmol), cyclopentylamine (267mg, 3.1mmol) and ethanol (10mL) was stirred at reflux for 9 hours. The solution was allowed to cool to room temperature before the addition of 2mL of 1.0N NaOH concentration of the solution afforded the crude product which was purified by elution from a silica gel column using 5% methanol in chloroform (0.430g, 68.4%). Crystallisation of such a sample from ethanol-acetonitrile gave an off-white powder, Mp. 143-146°C, 'H-NMR (Me₂SO-d₆) δ 7.60 (s, 1H, purine H-8), 7.00 (br,m, 1H, NH) 6.10 and 5.86 (m, 2H, CH=CH), 5.77 (br s, 2H, NH₂) 5.39 (m, 1H, NCH cyclopentene), 4.76 (br s, 1H, OH), 4.53 (br m, 1H, NCH), 3.44 (m, 2H, OCH₂), 2.87 (m, 1H, CH) 2.62-2.54 (m, 1H, 0.5 CH₂ cyclopentene ring), 1.89 (br m, 2H, cyclopentyl CH₂), 1.70-1.48 (br, m, 7H, 0.5 CH₂ cyclopentene ring, 3CH₂).

| | | | |
|---|---|---|---|
| Anal. calc. for C₁₆H₂₂N₆0.0.25H₂O: | C,60.26; | H,7.11; | N,26.35. |
| Found: | C,60.43; | H,7.16; | N,26.27 |
| | C,60.37; | H,7.17; | N,26.25. |

### Example 19

### (±)-cis-4-(2-Amino-6-allylamino-9H-purin-9-yl)-2-cyclopentene-1-methanol

(±)-(1α,4α)-4-(2-Amino-6-chloro-9H-purin-9-yl)-2-cyclopentene-1-methanol (0.531g, 2mmol) from Example 4, allylamine (1.014g, 17.8mmol) and ethanol (5mL) were stirred at reflux for 2.75 hours. The solution was allowed to cool to room temperature before the addition of 2.0mL of 1N NaOH, Evaporation afforded the crude product which was purified by elution from a silica gel column with 5% methanol - chloroform (0.36g, 63%). Crystallisation of such a sample from ethanol yielded an off white powder, Mp. 181-183°C; 'H-NMR (Me₂SO-d₆) δ 7.58 (s, 1H, purine H.8), 7.28 (br s, 1H, NH), 6.11-6.06 (m, 1H, 0.5CH=CH) 5.98-5.82 (m overlapping br s at 5.86, 4H 0.5CH=CH, CH=, NH₂), 5.37 (m, 1H, NCH), 5.16-4.98 (m,2H, =CH₂), 4.72 (t,J=5.1Hz, 1H, OH) 4.07 (br m, 2H, NCH₂), 3.42 (m, 2H, OCH₂) 2.84 (br m, 1H, CH), 2.65-2.54 and 1.62-1.57 (m, 2H, cyclopentyl CH₂).

| | | | |
|---|---|---|---|
| Anal. calc. for C₁₄H₁₈N₆O: | C,58.73; | H,6.34; | N,29.35. |
| Found: | C,58.47; | H,6.42; | N,29.19. |

### Example 20

### (±)-cis-4-(2-Amino-6-morpholino-9H-purin-9-yl)-2-cyclopentene-1-methanol

A solution of (±)-cis-4-(-2-Amino-6-chloro-9H-purin-9-yl)-2-cyclopentene-1-methanol (0.531g, 2.00mmol) from Example 4 and morpholine (0.526g, 6.04mmol) in ethanol (6mL) was stirred at reflux for 1 hour. The reaction mixture was allowed to cool to room temperature before the addition of 2mL of 1.0N NaOH. Concentration of the mixture afforded the crude product which was purified by elution from a silica gel column using 5% methanol in chloroform (0.62g, 98%). Crystallisation of such a sample from ethanol-water gave a white powder; Mp 165-167°C; 'H-NMR (Me₂SO-d₆) δ 7.62 (s, 1H, purine H-8), 6.09 (m, 1H 0.5CH=CH), 5.90-5.82 (m overlapping br s at 5.86, 3H, 0.5CH=CH, NH₂), 5.39 (m, 1H, NCH) 4.72 (t,J=5.0Hz, 1H, OH) 4.09 and 3.64 (br, m, 8H, 4CH₂ on morpholine ring), 3.42 (m, 2H, OCH₂), 2.84 (br m, 1H, CH), 2.65-2.54 and 1.59-1.47 (m, 2H, CH₂).

| | | | |
|---|---|---|---|
| Anal. calc. for C₁₅H₂₀N₆O₂: | C,56.95; | H,6.37; | N,26.57. |
| Found: | C,57.03; | H,6.41; | N,26.48. |

### Example 21

### (±)-cis-4-(2-Amino-6-benzylamino-9H-purin-9-yl)-2-cyclopentene-1-methanol

A solution of (±)-cis-4-(-2-Amino-6-chloro-9H)-purin-9-yl)-2-cyclopentene-1-methanol (0.531g, 2.00mmol) from Example 4, benzylamine (0.214g, 2.00mmol) and triethylamine (1.717g, 17mmol) in the ethanol (6mL) was stirred at reflux for 4 hours. The reaction mixture was allowed to cool to room temperature before the addition of 2mL of 1.0N NaOH, Concentration of the mixture afforded the crude product which was eluted from a silica gel column with 5% methanol-chloroform. Crystallisation of such a sample from ethanol-water yielded a white powder (0.386g, 57%); Mp.174-176°C; 'H-NMR (Me₂SO-d₆) δ 7.80-7.65 (br s, 1H, NH), 7.60 (s, 1H, purine H-8), 6.08 (m, 1H 0.5CH=CH), 5.86-5.82 (m overlapping br s, 3H 0.5CH=CH, NH₂), 5.35 (br m, 1H, NCH), 4.73 (t,J=4.9Hz, overlapping br s at 4.65, 3H, OH, NCH₂) 3.43 (m, 2H, OCH₂), 2.85-2.81 (br m 1H, CH), 2.65-2.54 and 1.63-1.50 (m, 2H, CH₂).

| | | | |
|---|---|---|---|
| Anal. calc. for C₁₈H₂₀N₆O: | C,64.27; | H,5.99; | N,24.98. |
| Found: | C,64.35; | H,6.02; | N,24.92. |

### Example 22

### (±)-cis-4-(2-Amino-6-(2-methoxyethoxy-9H-purin-9-yl)-2-cyclopentene-1-methanol

A flask was charged with sodium hydride (60% oil dispersion, 298mg, 7.45mmol) which was then washed with hexanes before the addition of methoxyethanol (15mL), (±)-cis-4-(2-Amino-6-chloro-9H-purin-9-yl)-2-cyclopentene-1-methanol (0.531g, 2mmol) from Example 4 was added and the solution was stirred at 100°C for 1 hour and then neutralized by the addition of 1.0N HCl. Concentration of the solution afforded the crude product which was purified by elution from a silica gel column with 5% methanol-chloroform (416mg, 68%). Crystallisation from ethanol gave an off-white powder, Mp.121-123°C; 'H-NMR (Me₂SO-d₆) δ 7.78 (S, 1H, purine H8), 6.39 (br s, 2H, NH₂), 6.14 and 5.90 (m, 2H, CH=CH), 5.44 (br m, 1H, NCH), 4.72 (t,J=4.6Hz, 1H, OH) 4.72 and 3.69 (m, 4H, OCH₂ CH₂O), 3.45 (m, 2H, OCH₂), 3.31 (s, overlapping with H₂O, OCH₃), 2.88 (br m, 1H, CH), 2.67-2.60 and 1.65-1.58 (m, 2H, CH₂).

| | | | |
|---|---|---|---|
| Anal. calc. for C₁₄H₁₉N₅O₃: | C,55.07; | H,6.27; | N,22.94. |
| Found: | C,55.18; | H,6.33; | N,22.95. |

### Example 23

### (±)-cis-4-(2-Amino-6-propylamino-9H-purin-9-yl)-2-cyclopentene-1-methanol

A solution of (±)-cis-4-(-2-Amino-6-chloro-9H-purin-9-yl)-2-cyclopentene-1-methanol (0.531g, 2.00mmol) from Example 4 and propylamine (1.12g, 18.19mmol) in 8mL of ethanol was stirred at reflux for 2 hours. The solution was allowed to cool to room temperature before the addition of 2mL of 1.0N NaOH. Concentration of the solution afforded the crude product which was purified by elution from a silica gel column with 5% methanol-chloroform (0.46g, 80%). Crystallisation of such a sample from ethanol-ethyl acetate yielded a white powder, Mp.138-140°C; 'H-NMR (Me₂SO-d₆) δ 7.57 (S, 1H, purine H-8) 7.20-7.05 (br s, 1H, NH), 6.11-6.06 and 5.86-5.82 (both m, 2H, CH=CH), 5.78-5.70 (br s, 2H, NH₂), 5.39 (m,1H, NCH), 4.73 (t,J=5.3, 1H, OH), 3.45-3.28 (br m, overlapping H₂O, OCH₂, NCH₂) 2.93-2.78 (br m, 1H CH), 2.65-2.54 (m, 1H 0.5 cyclopentyl CH₂), 1.62-1.58 (m, 3H, 0.5 cyclopentyl CH₂, CH₂) 0.86 (t, J=7.4Hz, 3H, CH₃).

| | | | |
|---|---|---|---|
| Anal. calc. for C₁₄H₂₀N₆O: | C,58.32; | H,6.99; | N,29.15. |
| Found: | C,58.38; | H,7,02; | N,29.10. |

### Example 24

### (±)-cis-4-(2-Amino-6-anilino-9H-purin-9-yl)-2-cyclopentene-1-methanol

A solution of (±)-cis-4-(2-Amino-6-chloro-9H-purin-9-yl)-2-cyclopentene-1-methanol (0.550g, 2.07mmol) from Example 4 and aniline (0.965g, 10.35mmol) in 10mL of methoxyethanol was stirred at 95°C for 1 hour. The reaction mixture was allowed to cool to room temperature before the addition of 2.05mL of 1N NaOH. Concentration of the solution afforded the crude product which was purified by elution from a silica gel column with 5% methanol-chloroform (0.60g, 90%). Crystallisation of such a sample from methanol-acetonitrile gave an off-white powder Mp.177-179°C; 'H-NMR (Me₂SO-d₆ δ 9.32 (s, 1H, NH), 8.00 (d,J=7.8Hz, 2H, C₆H₅), 7.74 (s, 1H, purine H-8), 7.25 (m, 2H, C₆H₅), 6.94 (m, 1H, C₆H₅), 6.18-6.05 (br m, 3H, NH₂, 0.5CH=CH), 5.88 (m, 1H, 0.5CH=CH), 5.50-5.35 (br m, 1H, NCH), 4.74 (m, 1H, OH),3.45 (m, 2H, CH₂O), 2.97-2.80 (br m, 1H, CH), 2.65 and 1.61 (2m, 2H, CH₂).

| | | | |
|---|---|---|---|
| Anal. calc. for C₁₇H₁₈N₆O: | C,63.34; | H,5.63; | N,26.07. |
| Found: | C,63.26; | H,5.67; | N,26.01 |

### Example 25

### (±)-cis-4-(2-Amino-6-methylamino-9H-purin-9-yl)-2-cyclopentene-1-methanol

(±)-cis-4-(2-Amino-6-chloro-9H-purin-9-yl)-2-cyclopentene-1-methanol (0.400g, 1.5mmol) from Example 4 and methylamine (40% aqueous solution, 25mL) were stirred at 60°C for 0.5 hours. The solution was allowed to cool to room temperature before the addition of 1.5mL of 1.5N NaOH. Evaporation left the crude product which was purified by elution from a silica gel column with 5% methanol-chloroform. The resulting solid was slurried in acetonitrile to yield a pale yellow powder (0.274g, 70%); Mp.221-223°C; 'H-NMR (Me₂SO-d₆) δ 7.57 (s, 1H, purine H-8), 7.12 (br s, 1H, NH), 6.11-6.06 (m, 1H, 0.5CH=CH), 5.87-5.80 (m, overlapping br s at 5.80, total 3H 0.5CH=CH, NH₂), 5.40-5.33 (m, 1H, CHN), 4.76-4.70 (m, 1H, OH), 3.43 (m , 2H, CH₂O) 2.95-2.77 (br s, 4H, CH₃, CH), 2.65-2.54 and 1.62-1.50 (both m, 2H, CH₂).

| | | | |
|---|---|---|---|
| Anal. calc. for C₁₂H₁₆N₆O: | C,55.37; | H,6.20; | N,32.29. |
| Found: | C,55.28; | H,6.24; | N,32.19. |

### Example 26

### (±)-cis-4-(2-Amino-6-dimethylamino-9H-purin-9-yl)-2-cyclopentene-1-methanol

(±)-cis-4-(2-Amino-6-chloro-9H-purin-9-yl)-2-cyclopentene-1-methanol (0.400g, 1.5mmol) from Example 4 and dimethylamine (25% aqueous solution, 20mL) was stirred at 80°C for 0.5 hours. The reaction mixture was allowed to cool to room temperature before the addition of 1.5mL of 1N NaOH. Concentration of the solution afforded the crude product which was purified by elution from a silica gel column with 5% methanol-chloroform (0.310g, 75%). Crystallisation of such a sample from ethanol-water gave an off-white powder Mp.173-174°C; 'H-NMR (Me₂SO-d₆) δ 7.60 (s, 1H, purine H-8), 6.10 and 5.84 (both m, 2H, CH=CH), 5.78 (s, 2H, NH₂), 5.39 (m, 1H, CHN), 4.72 (m, 1H, OH), 3.42 (m, OCH₂ overlapping with H₂O, NMe₂), 3.33 and 3.31 (both s, N(CH₃)₂ overlapping with H₂O, OCH₂), 2.90-2.78 (m, 1H, CH), 2.65-2.53 and 1.59-1.47 (both m, 2H, CH₂).

| | | | |
|---|---|---|---|
| Anal. calc. for C₁₃H₁₈N₆O: | C,56.92; | H, 6.61; | N,30.63. |
| Found: | C,56.93; | H,6.64; | N,30.56 |

### Example 27

### (±)-cis-4-(2-Amino-6-propoxy-9H-purin-9-yl)-2-cyclopentene-1-methanol

A flask was charged with sodium hydride (60% oil dispersion, 158mg, 3mMol) which was then washed with hexanes before the addition of n-propanol (25mL). (±)-cis-4-(2-Amino-6-chloro-9H-purin-9-yl)-2-cyclopentene-1-methanol from Example 4 (400mg, 1.5mmol) was added and the solution was stirred at 80°C for 2 hours and the neutralized by the addition of 1.0N HCl. Concentration of the solution afforded the crude product which was purified by elution from a silica gel column with 2% methanol-chloroform (350mg, 81%). Crystallisation of such a sample from ethanol-water gave an off-white powder, Mp.97-99°C; 'H-NMR (Me₂SO-d₆) δ 7.74 (s, 1H, purine, H-8), 6.34 (s, 2H, NH₂), 6.11 and 5.85 (both m, 2H, CH=CH), 5.41 (m, 1H, CHN), 4.71 (m, 1H, OH), 4.34 (t,J=6.8, 2H, OCH₂), 3.44 (m, 2H, CH₂ OH), 2.85 (m, 1H, CH), 2.60 (m, 1H, 0.5 CH₂), 1.74 (m, 2H, CH₂CH₃) 1.61 (m, 1H, 0.5 CH₂, 0.95 (t,J=7,4, 3H, CH₃).

| | | | |
|---|---|---|---|
| Anal. calc for C₁₄N₁₉N₅O₂.0.35H₂O: | C,56.88; | H,6.72; | N,23.69. |
| Found: | C,57.00; | H,6.78; | N,23.61 |
| | C,56.93; | H,6.81; | N,23.59 |

### Example 28

### (±)-cis-4-(2-Amino-6-((2-hydroxyethyl)amino)-9H-purin-9-yl)-2-cyclopentene-1-methanol

A flask charged with (±)-cis-4-(2-Amino-6-chloro-9H-purin-9-yl)-2-cyclopentene-1-methanol (0.53g, 2.00mmol) from Example 4, triethylamine (0.92g, 9.10mmol), ethanolamine (0.172g, 2.82mmol) and methoxyethanol (6mL) was stirred at reflux for 2 hours. The solution was allowed to cool to room temperature before the addition of 2mL of 1.0N NaOH. Concentration of the solution left the crude product which was purified by elution from a silica gel column with 10% methanol chloroform (0.430g, 74%). Crystallisation of such a sample from ethanol-water gave a white powder, Mp.150-152°C; 'H-NMR (Me₂SO-d₆) δ 7.57 (s, 1H, purine H-8), 7.00-6.87 (br s, 1H, NH), 6.11-6.06 (m, 1H, 0.5CH=CH), 5.87- 5.80 (m, overlapping br s, 3H 0.5 CH=CH, NH₂), 5.37 (m, 1H, CHN), 4.72 (two t, 2H, 20H) 3.53-3.40 (m, 6H, 2 OCH₂), 2.87-2.80 (br m, 1H, CH) 2.65-2.54 and 1.62-1.50 (2m, 2H, CH₂).

| | | | |
|---|---|---|---|
| Anal. calc. for C₁₃H₁₈N₆O₂: | C,53.78; | H,6.25; | N,28.95 |
| Found: | C,53.89; | H,6.33; | N,28.90 |

### Example 29

### (±)-cis-4-(2-Amino-6-(cyclopropylmethylamino)-9H-purin-9-yl)-2-cyclopentene-1-methanol

(±)-cis-4-(2-Amino-6-chloro-9H-purin-9-yl)-2-cyclopentene-1-methanol (0.53g, 2mmol) from Example 4, N-methyl-N-cyclopropylamine (0.8477g, 12mmol) and methanol (20mL) were placed in a Parr bomb and heated to 62°C for 5 hours. The solution was concentrated and then diluted with ethanol before being brought to pH12 by the addition of 1.0N NaOH. This solution was concentrated and the residue was purified by elution from a silica gel column with 3% Methanol-chloroform (0.547g, 91.2%). Crystallisation of such a sample from water-ethanol yielded a white powder, Mp. 130-131°C; 'H-NMR (Me₂SO-d₆): δ7.61(s,1H,purine H-8), 6.10(m,1H,CH=), 5.84(m,1H,CH=), 5.7(brs,2H,NH₂), 5.40(M,1H,CHN), 4.70(brt,1H,OH), 3.43(m,2H,CH₂OH) 3.24(brs,4H,CH₃,NCH cyclopropyl), 2.85(m,1H,CH), 2.66-2.57 and 1.61-1.51(m,2H₂CH cyclopentene), 0.90-0.65(m,4H,2CH₂ cyclopropane).

| | | | |
|---|---|---|---|
| Anal. calc. C₁₅H₂₀N₆O.0.5H₂O: | C,58.24; | H,6.84; | N,27.16. |
| Found: | C,58.15; | H,6.86; | N,27.14. |

### Example 30

### (±)-cis-4-[2-Amino-6-((R)sec-butoxyl)-9H-purin-9-yl]-2-cyclopentene-1-methanol

A flask was charged with sodium hydride (60% oil dispersion, 300mg, 7.5mmol) which was washed with hexanes before the addition of (±)-cis-4-(2-amino-6-chloro-9H-purin-9-yl)-2-cyclopentene-1-methanol from Example 4 (800mg, 3mmol) in ∼10mL of R-(-)-2-butanol. The solution was stirred at room temperature for 3 hours then at 60°C (oil bath) for 1 hour. The solution was allowed to cool to room temperature before neutralization with 1N HCl. The solution was concentrated and the residue chromatographed on silica gel. Title compound was eluted with 5% methanol-chloroform (0.81g, 89%). Crystallisation of such a sample from ethanol-acetonitrile yielded an off-white powder, Mp. 159-161°C; 'H-NMR (Me₂SO-d₆) δ 7.72(s,1H,purine H.8), 6.30(br s,2H, NH₂), 6.10 and 5.86(m,2H,HC=CH), 5.44-5.28 (m, 2H, NCH, OCH), 4.71(t, J=5.0, 1, OH), 3.42(m, 2, OCH₂), 2.85(m, 1, CH), 2.60(m 1, 0.5 CH₂ cyclopentene), 1.61(m,3H 0.5 CH₂ cyclopentene, CH₂) 1.27(d,J=6.0,3H,CHCH₃), 0.89(t,J=7.4,3H,CH₃).

| | | | |
|---|---|---|---|
| Anal. calc. C₁₅H₂₁N₅O₂: | C,59.39; | H,6.98; | N,23.09. |
| Found: | C,59.28; | H,7.01; | N,23.02. |

### Example 31

### (±)-cis-4-(2-Amino-6-cyclobutylamino-9H-purin-9-yl)-2-cyclopentene-1-methanol

A solution of (±)-cis-4-(-2-Amino-6-chloro-9H-purin-9-yl)-2-cyclopentene-1-methanol from Example 4 (0.53g, 2mmol) and cyclobutylamine (1.387g, 19.5mmol) in methanol (15mL) was heated at 70-75°C (oil bath) for 29 hours. After cooling to room temperature, 2mL of 1.0N NaOH was added. The solution was concentrated and the residue chromatographed on silica gel. Title compound was eluted with 5% methanol-chloroform as colourless foam which solidified to white powder in acetonitrile (454mg, 75.7%), Mp. 181-183°C; 'H-NMR (DMSO-d₆) δ 7.59(s,1H, purine H.8), 7.38(br m,1H,NH), 6.10(m,1H 0.5 HC=CH) 5.84 (m overlapping br s, at 5.76, 3H, 0.5 CH=CH, NH₂) 5.36 (m,1H,NCH cyclopentene), 4.73(t, overlapping br m, J=5.2,2H,OH,NCH cyclobutane), 3.42(m,2H,OCH₂), 2.83(br m, 1H,CH), 2.55 (m overlapping with DMSO, 1/2 CH₂ cyclopentene), 2.20-1.95(br m, 4H, 2CH₂ cyclobutane), 1.58(m,3, CH₂ cyclobutane, 0.5 CH₂ cyclopentene).

| | | | |
|---|---|---|---|
| Anal. calc. C₁₅H₂₀N₆O: | C,59.98; | H,6.71; | N,27.98. |
| Found: | C,60.05; | H,6.73; | N,27.91. |

### Example 32

### (±)-cis[4-[2-Amino-6-(cyclopropylamino)-9H-purin-9-yl]-2-cyclopenten-1-yl] methylacetate

A solution of (±)-cis-4-(2-amino-6-cyclopropylamino-9H-purin-9-yl)-2-cyclopentene-1-methanol from Example 4 (400mg, 1.5mmol), acetic anhydride (228mg, 2.2mmol),4-N,N-dimethylaminopyridine (8.4mg, 6.9 10⁻² mmol) and dry N,N-dimethylformamide (12mL) were stirred at room temperature overnight. The solution was concentrated under high vacuum and the residues were placed on a silica gel column which was eluted with 5% methanol-chloroform, (240mg, 48.7%). The title compound was diluted in ethanol and foamed under high vacuum. 'H-NMR: (DMSO-d₆) δ 7.52 (s,1H, purine H8), 7.28(d,J=4.5,1H,NH), 6.07 and 5.94 (m,2H,HC=CH) 5.81(br,2H,NH₂), 5.39(br m,1H,NCH), 4.06(m, 2H,OCH₂₎ 3.02(br m, 2H, CH, NCH cyclopropane), 2.65 (m,1H 0.5 CH₂ cyclopentene) 1.98(s,3H,CH₃), 1.56(m,1H, 0.5 CH₂ cyclopentene), 0.61(m,4H, 2CH₂ cyclopropane).

| | | | |
|---|---|---|---|
| Anal. calc. C₁₆H₂₀N₆O₂.0.4H₂O.0.15EtOH: | C,57.16; | H,6.39; | N,24.54. |
| Found: | C,56.88; | H,6.32; | N,24.81 |
| | C,56.82; | H,6.32; | N,24.78. |

### Example 33

### (±)-cis-4-(2-Amino-6-butylamino-9H-purine-9-yl)-2-cyclopentene-1-methanol

A solution of (±)-cis-4-(2-amino-6-chloro-9H-purin-9-yl)-2-cyclopentene-1-methanol from Example 4 (0.408g, 1.5mmol): butylamine (0.549g, 7.5mmol, 5 eqv.) and ethanol (10mL) was stirred at reflux for 3 hours. The solution was allowed to cool to room temperature before the addition of 1.5 mL of 1.0N NaOH. The solution was concentrated and the residues were purified by elution from a silica gel column with 5% methanol-chloroform (0.440g, 97%). The title compound was dissolved in hot acetonitrile then cooled to produce a white powder. Mp. 116-118°C; 'H-NMR (Me₂SO.d₆) δ 7.57 (s, 1H, purine H.8) 7.12(brm,1H,NH), 6.09 and 5.85 (m,2H,HC=CH), 5.75(br s,2H,NH₂) 5.36(br m,1H,NCH), 4.74(t,J=5.3,1H,OH), 3.42(m,4H,OCH₂,NCH₂), 2.84(br m,1H,CH), 2.58(m,1H, 0.5 CH₂ cyclopentene), 1.52 (m,3H 0.5 CH₂ cyclopentene, CH₂ butyl), 1.30 (m,2H,CH₂ butyl), 0.87(t,J=7.2, 3H, CH₃).

| | | | |
|---|---|---|---|
| Anal. calc. C₁₅H₂₂N₆O: | C,59.58; | H,7.34; | N,27.80. |
| Found: | C,59.44; | H,7.38; | N,27.79. |

### Example 34

### cis-4-(2-Amino-6-((s)-sec-butoxy)-9H-purin-9-yl)-2-cyclopentene-1-methanol

A flask was charged with sodium hydride (60% oil dispersion, 300mg, 7.5mmol) which was washed with hexanes before the addition of (±)-cis-4-(2-amino-6-chloro-9H-purin-9-yl)-2-cyclopentene-1-methanol from Example 4 (800mg, 3mmol) in -(t)-2-butanol (10mL). The solution was stirred at room temperature for 6 hours before neutralization with 1N HCl. The solution was concentrated and the residue was placed on a silica gel column. Title compound was eluted with 5% methanol-chloroform (0.29g, 32%). Crystallisation of such a sample from ethanol: acetonitrile yielded an off-white powder, Mp. 159-162°C; 'H-NMR (Me₂SO-d₆) δ 7.72(s,1H, purine H.8), 6.30(br s, 2H, NH₂) 6.10 and 5.86 (m,2H,HC=CH), 5.44-5.28 (m,2H,NCH,OCH) 4.71(t,J=5.0,1H,OH), 3.42(m,2H,OCH₂), 2.85(m,1H,CH), 2.60(m,1H, 0.5 CH₂ cyclopentene), 1.61(m, 3H, 0.5 CH₂ cyclopentene, CH₂), 1.27(d,J=6.0, 3H, CH, CH₃), 0.89(t,J=7.4, 3H, CH₃).

| | | | |
|---|---|---|---|
| Anal. calc. C₁₅H₂₁N₅O₂.0.25 H₂O: | C,58.52; | H,7.04; | N,22.75. |
| Found: | C,58.59; | H,6.94; | N,22.79. |

### Example 35

### (±)(cis-4-(2-Amino-6-[(6-hydroxyhexyl)thio]-9H-purin-9-yl)-2-cyclopentene-1-methanol

To a solution of (±)-cis-2-amino-1,9-dihydro-9-[(4-hydroxymethyl)-2-cyclopenten-1-yl]-6H-purin-6-thione-HCl, from Example 9 (0.50g, 1.45 mmol) in 1N NaOH (2.9mL) was added 6-bromo-1-hexanol (0.321g, 1.77 mmol) in dioxane (1mL). The solution was stirred at room temperature under nitrogen for 5 hours during which time additional 6-bromo-1-hexanol (0.096g, 0.53 mMol) and 1N NaOH (0.53ml) were added. The solution was evaporated to remove dioxane and the aqueous layer was extracted with 3 x 25ml chloroform Combined chloroform extracts were dried (MgSO₄). Solvent was evaporated and the residual oil was chromatographed on silica gel. The title compound was eluted with 10% methanol-chloroform; white solid was formed after crystallisation from acetonitrile (0.469g, 89%), Mp. 129-130°C; 'H-NMR (DMSO-d₆) δ: 7.84 (s,1,H-8), 6.46(br s,2,NH₂), 6.15 and 5.90 (2m,2,CH=CH), 5.45(br m,1,CH-N), 4.73(t,J=5.4,1,OH) 4.36(t,J=5.2,1,(CH₂)₆-OH), 3.50-3.20(all m, overlapping H₂O,2CH₂-O CH₂S), 2.90(br m,1,CH), 2.70-2.55(m,1, 0.5 CH₂), 1.70-1.50(m,3,S-CH₂-CH₂) and 0.5 CH₂), 1.50-1.20(br m,6,3CH₂S).

| | | | | |
|---|---|---|---|---|
| Anal calc. C₁₇H₂₅N₅O₂S: | C,56.18; | H,6.93; | N,19.27; | S,8.82. |
| Found: | C,56.09; | H,6.93; | N,19.22; | S,8.90. |

### Example 36

### (±)-cis-4-(2-Amino-6-(isopropylamino)-9H-purin-1-yl)-2-cyclopenten-1-methanol

(±)-cis-4-(2-Amino-6-chloro-9H-purin-9-yl)-2-cyclopentene-1-methanol from Example 4 (0.545g, 2mmol), isopropylamine (2.36g, 40 mmol) and methanol (15mL) were placed in a Parr bomb and heated at 65-70°C for 12 hours. 1N NaOH (2mL) was added and the solution evaporated to dryness. The residue was dried by evaporation of ethanol and chromatographed on silica gel. The title compound was eluted with 10% methanol-chloroform; white solid was formed after crystallisation from acetonitrile (0.463g, 80%), Mp. 153-155°C; 'H-NMR (DMSO-d₆) δ: 7.60(s,1,H8), 6.90(br m,1,NH), 6.10 and 5.85(2m,2,CH=CH), 5.70(br s,2,NH₂), 5.40(br m,1,CH-N), 4.75(tJ=5,O,1,OH), 4.60-4.40(br m, 1,CH-NH), 3.45(m,2,CH₂-O), 2.85(br m,1,CH), 2.65-2.55(m,1 0.5 CH₂) 1.65-1.55(m,1, 0.5 CH₂), 1.17(d,J=6.5,6,2CH₃).

| | | | |
|---|---|---|---|
| Anal. calc. C₁₄H₂₀H₆O: | C,58.32; | H,6.99; | N,29.15. |
| Found: | C,58.42; | H,7.00; | N,29.23. |

### Example 37

### (±)-cis-4-(2-Amino-6-(butylthio)-9H-purin-9-yl)-2-cyclopentene-1-methanol

To a solution of (±)-cis-2-amino-1,9-dihydro-9-[(4-hydroxymethyl)-2-cyclopenten-1-yl]-6H-purin-6-thione from Example 9 (0.50g, 1.89mmol) in 1N NaOH (1.89mL) was added butyliodide (0.348g, 1.89mmol), in dioxane (5mL). The solution was stirred for 5 hours under nitrogen at 25°C and extracted with CHCl₃ (3 x 100mL). The combined chloroform extracts were dried (MgSO₄), solvent evaporated and the residual oil crystallised from acetonitrile to give title compound as yellow powder (0.491g, 81%), Mp. 120-123°C; 'H-NMR (DMSO-d₆) δ: 7.82(s,1H-8), 6.43 (br s,2,NH₂), 6.15 and 5.85(2m,2,CH=CH), 5.40(m,1,CH-N), 4.70(m,1,OH), 3.43(m,2,CH₂-O), 3.25(t,J=7.4, overlapping H₂O, S-CH₂), 2.85(br m,1,CH), 2.70-2.55(m,1, 0.5 CH₂), 1.70-1.30(m,5 - CH₂-CH₂- + 1/2 CH₂), 0.89(t,J=7.2,3,CH₃).

| | | | | |
|---|---|---|---|---|
| Anal. calc. C₁₅H₂₁N₅OS: | C,56.40; | H,6.62; | N,21.93 | S,10.04. |
| Found: | C,56.31; | H,6.64: | N,21.89; | S,10.02. |

### Example 38

### (±)-cis-4-(2-Amino-6-(isobutylthio)-9H-purin-9-yl)-2-cyclopentene-1-methanol

To a solution of (±)-cis-2-amino-1,9-dihydro-9-[(4-hydroxymethyl)-2-cyclopenten-1-yl]-6H-purine-6-thione-HCl from Example 9 (0.50g, 1.45mmol), in 1N NaOH (2.9ml) was added 1-iodo-2-methylpropane (0.326g, 1.77mmol) in dioxane (1mL). The reaction was stirred at room temperature under nitrogen for 24 hours during which time additional 1-iodo-2-methyl propane (0.130g, 0.71mmol) and 1N NaOH (0.71ml) were added. The solution was then evaporated to remove dioxane and the aqueous layer extracted with 3 x 25ml chloroform. The combined chloroform extracts were dried (MgSO₄), solvent evaporated and the residual oil chromatographed on silica gel. The title compound was eluted with 10% methanol-chloroform; white powder was formed after crystallisation from acetonitrile (0.345g, 75%), Mp. 127-129°C; 'H-NMR (DMSO-d₆) δ: 7.82 (s,1,H-8), 6.44(br s,2,NH₂), 6.15 and 5.85 (2m,2,CH=CH), 5.45(br m,1,CH-N), 4.71(m,1,OH), 3.45(m,2,CH₂-O), 3.18 (d,J=6.6,2,S-CH₂), 2.85(br m,1,CH), 2.70-2.50(m, overlapping solvent, 0.5 CH₂), 2.0-1.80(m,1,Me₂CH), 1.70-1.50(m,1, 0.5 CH₂), 0.983 (d,J=6.6,6,2CH₃).

| | | | | |
|---|---|---|---|---|
| Anal. calc. C₁₅H₂₁N₅OS: | C,56.40; | H,6.62; | N,21.93; | S,10.04. |
| Found: | C,56.48; | H,6.61; | N,21.93; | S,10.10. |

### Example 39

### (±)-cis-4-(2-Amino-6-(cyclopentylthio)-9H-purin-9-yl)-2-cyclopentene-1-methanol

To a solution of (±)-cis-2-amino-1,9-dihydro-9-[(4-hydroxymethyl)-2-cyclopentene-1-yl]-6H-purin-6-thione from Example 9 (0.50g, 1.89mmol) in 1N NaOH (1.89ml) was added cyclopentylbromide (0.282g, 1.89mmol) in dioxane (1mL). The solution was stirred under nitrogen at room temperature for 24 hours during which time additional cyclopentyl bromide (0.846g, 5.67mmol) and 1N NaOH (5.67ml) were added. The solution was evaporated to remove dioxane and the aqueous layer extracted with 3 x 50ml of chloroform. The combined extracts were dried (MgSO₄), solvent evaporated and the residual oil chromatographed on silica gel. The title compound was eluted with 10% methanol-chloroform and solidified in acetonitrile to give title compound as yellow powder (0.310g, 50%), Mp. 167-169°C; 'H-NMR (DMSO-d₆) δ: 7.83(S,1,H-8), 6.43(brs,2,NH₂), 6.13 and 5.87(2M,2,CH=CH), 5.42(br m,1,CH-N), 4.72(t,J=5.3,1,OH), 4.30(m,1,S-CH), 3.44(m,2,CH₂-O), 2.85(br m,1,CH), 2.70-2.55(m,1, 0.5 CH₂), 2.30-2.15(brm,2,2CH), 1.80-1.50(m,7,2CH₂ plus 3/2 CH₂).

| | | | | |
|---|---|---|---|---|
| Anal. calc. C₁₆H₂₁N₅OS: | C,57.98; | H,6.39; | N,21.13; | S,9.67. |
| Found: | C,57.82; | H,6.42; | N,21.10; | S,9.57. |

### Example 40

### (±)-cis-4-(2-Amino-6-[(cyclopropylmethyl)thio]-9H-purin-9-yl)-2-cyclopentene-1-methanol

To a solution of (±)-cis-2-amino-1,9-dihydro-9-[(4-hydroxymethyl)-2-cyclopenten-1-yl]-6H-purin-6-thione-HCl from Example 9 (0.50g, 1.45mmol), 1N NaOH (2.9ml) was added bromomethyl cyclopropane (0.239g, 1.77mmol) in dioxane (1ml). The solution stirred under nitrogen at room temperature for 2 hours and was then extracted with 3 x 50ml, chloroform. The combined chloroform extracts were dried (MgSO₄), solvent evaporated and residual oil chromatographed on silica gel. The title compound was eluted with 10% methanol-chloroform; white solid was formed after crystallisation from acetonitrile. (0.350g, 76%), Mp. 125-127°C; 'H-NMR (DMSO-d₆) δ: 7.82(S,1,H-8), 6.44(br s,2,NH₂), 6.10 and 5.85(2m,2,CH=CH), 5.40(br m,1,CH-N), 4.71(m,1,OH), 3.43(m,2,CH₂-O), 3.26(d,J=12.1, 2, S-CH₂), 2.85(br m,1, CH), 2.70-2.50(m, overlapping solvent, 0.5 CH₂), 1.70-1.50 (m, 1, 0.5 CH₂), 1.20.1.0(m,1,CH cyclopropyl), 0.55 and 0.35 (both m, 4, cyclopropyl CH₂).

| | | | | |
|---|---|---|---|---|
| Anal calc. C₁₅H₁₉N₅OS: | C,56.76; | H,6.03; | N,22.07; | S,10.10. |
| Found: | C,56.65; | H,6.05; | N,22.01; | S,10.19. |

### Example 41

### (±)-cis-4-(2-Amino-6-((6-hydroxyhexyl)amino)9H-purin-9-yl-2-cyclopentene-1-methanol

A solution of (±)-(cis)-4-(2-amino-6-chloro-9H-purin-9-yl)-2-cyclopentene-1-methanol from Example 4 (0.544g 2mmol), triethylamine (0.607g, 6mmol) and 6-amino-1-hexanol (0.234g, 2mmol) in ethanol (5mL) was refluxed under nitrogen for 32 hours. During this time additional 6-amino-1-hexanol (0.117g, 1mmol) was added. 1N NaOH (2mL) was then added and the solution allowed to stir for 30 minutes. The solution was concentrated under vacuum, and the residual oil was dried by evaporation of ethanol and chromatographed on silica gel. Title compound was eluted with 7% methanol-chloroform; white powder after crystallisation from acetonitrile (0.473g, 68%), Mp. 120-121°C; 'H-NMR(DMSO-d₆) δ: 7.57 (S,1,H-8), 7.10(br s,1,NH), 6.10 and 5.85 (2m,2,CH=CH), 5.74(br s,2,NH₂), 5.40(m,1,CH-N), 4.73(t,J=5.3,1,OH), 4.31(t,J=5.2, (CH₂)₆-OH), 3.50-3.30(all m, overlapping H₂O,2,CH₂-O and CH₂-N), 2.85(br m,1,CH), 2.70-2.50(m, overlapping solvent, 0.5 CH₂), 1.70-1.20(all m,9,4 CH₂ and 0.5 CH₂).

| | | | |
|---|---|---|---|
| Anal. calc. for C₁₇H₂₆N₆O₂: | C,58.94; | H,7.56; | N,24.26 |
| Found: | C,58.84; | H.7.60; | N,24.21 |

### Example 42

### (±)-cis-4-(2-Amino-6-(3-butenylthio)-9H-purin-9-yl)-2-cyclopentene-1-methanol

To a solution of (±)-cis-2-amino-1,9-dihydro-9[(4-hydroxymethyl)-2-cyclopentene-1-yl]-6H-purin-6-thione-HCl from Example 9 (0.50g, 1.45mmol) in 1N NaOH (2.9ml) was added 4-bromo-1-butene (0.196g, 1.45mmol) in dioxane (1ml). The solution was allowed to stir at room temperature for 5 hours during which time an additional 4-bromo-1-butene (0.196g, 1.45mmol) and 1N NaOH (1.45ml) were added. The solution was then evaporated to remove dioxane and aqueous layer extracted with chloroform (3 x 25ml). The combined chloroform extracts were dried (MgSO₄), solvent evaporated and residual oil chromatographed on silica gel. The title compound was eluted with 7% methanol-chloroform; foamed from ethanol under vacuum (0.410g, 85%), 'H-NMR-(DMSO-d₆) δ:7.82(s,1,H-8), 6.45(br s,2,NH₂), 6.10 and 5.85(2m, overlapping 6.0-5.75,m, total 3, CH=CH and CH=CH₂), 5.40(br m, 1,CH-N), 5.20-5.0(m,2, CH=CH₂), 4.71(t,J=5.3, 1,OH), 3.50-3.25 (2m, overlapping H₂O, CH₂Y-S,CH₂-O), 2.85(br m,1,CH), 2.70-2.30(2m, overlapping solvent, 0.5 CH₂ and S-CH₂ CH₂), 1.70-1.50(m,1, 0.5 CH₂).

| | | | | |
|---|---|---|---|---|
| Anal. calc. for C₁₅H₁₉N₅OS. 0.25H₂O-0.20 EtOH: | C,55.86; | H,6.30; | N,21.15; | S,9.68 |
| Found: | C,55.97; | H,6.19; | N,20.77; | S,10.02. |

### Example 43

### (±)-cis-[4-(2-Amino-6-(cyclopropylmethylamino)-9H-purin-9-yl]-2-cyclopentene-1-yl]-methyl acetate

A solution of (±)-cis-4-(2 amino-6-(cyclopropylmethylamino)-9H-purin-9-yl)-2-cyclopentene-1-methanol from Example 29 (0.30g, 1mmol), acetic anhydride (0.204g, 2mmol), N,N-dimethylaminopyridine (0.005g, 0.04mmol) in N,N-dimethylformamide (10mL) was stirred at room temperature under nitrogen overnight. H₂O (1mL was added and the solution allowed to stir an additional hour, then concentrated under high vaccum. The residual oil was partitioned between saturated sodium bicarbonate solution (5mL) and chloroform (3x50mL). The combined chloroform extracts were dried (MgSO₄), solvent evaporated and residue chromatographed on silica gel. Title compound was eluted with 4% methanol-chloroform; foamed from ethanol under high vacuum (0.330g, 93%); 'H-NMR(DMSO-d₆) 7.59(s,1,H-8), 6.10 and 5.90(2m, 2, CH=CH), 5.80(br s, 2, NH₂), 5.40(br m, 1,CH-N), 4.05(d,J=6.1, 2, OCH₂), 3.30-3.20(m, overlapping s at 3.23, total 4 CH-N-Me and CH₃), 3.10(br m,1,CH), 2.75-2.60(m,1,0.5 CH₂), 1.65-1.50(m,1, 0.5 CH₂).

| | | | |
|---|---|---|---|
| Anal. calc. for C₁₇H₂₂N₆O₂.0.45H₂O 0.5 EtOH; | C,58.21; | H,6.63; | N,23.82. |
| Found: | C,58.15,58.09; | H,6.60,6.61; | N,23.91,23.83. |

### Example 44

### (±)-cis-4-(2-Amino-6-(tert-butylamino)-9H-purin-9-yl)-2-cyclopentene-1-methanol

(±)-cis-4-(2-Amino-6-chloro-9H-purin-9-yl)-2-cyclopentene-1-methanol from Example 4 (0.544g, 2.0mmol) and tert-butylamine (15mL) were heated at 80°C in a Parr bomb for 28 hours. The resulting solution was concentrated and the residual oil was dried by evaporation of ethanol and chromatographed on silica gel. Title compound was eluted with 5% methanol-chloroform; crystallisation from acetonitrile gave white powder (0.392g, 65%), Mp. 161-163°C; 'H-NMR(DMSO-d₆) δ: 7.56 (s,1,H-8), 6.10(m, overalpping s at 6.03, total 2, =CH and NH), 5.85(m, overlapping s at 5.78, total 3, =CH and NH₂), 5.40(br m,1,CH-N), 4.72 (t,J=5.3,1,OH), 3.40(m,2,CH₂-O), 2.85(br m,1,CH), 2.70-2.50(m, overlapping solvent, 0.5 CH₂), 1.65-1.40 (m, overlapping s at 1.45, total 10, 0.5 CH₂ and 3 CH₃).

| | | | |
|---|---|---|---|
| Anal. calc. for C₁₅H₂₂N₆O: | C,59.58; | H,7.33; | N.27.79 |
| Found: | C,59.58, | H,7.35; | N,27.86. |

### Example 45

### (±)-9-[3-(Hydroxymethyl)-3-cyclopenten-1-yl)guanine

A. (±)-Methyl-4-acetamido-1-cyclopentene-1-carboxylate
(±)-Cis-methyl 4-acetamido-2-cyclopentene 1-carboxylate [S. Daluge and R. Vince, J.Org.Chem. 1978, 43, 2311] (3.90g, 21.3mmol) was dissolved in dry methanol (25mL) and added to a solution of sodium methoxide prepared from sodium (0.98g, 43 m.equiv.) and dry methanol (150mL). This solution was stirred at 25°C for 2.0 hours and then neutralised with 1N hydrochloric acid. The solution was concentrated to 40mL and extracted with chloroform (3x75mL). The chloroform was dried (MgSO₄) and evaporated to colourless glass (3.9g). Chromatography on silica gel with 1-2% methanol-chloroform gave title compound as white crystals (4.33g, 82%); Mp. 72-74°C [Lit.: Can.J.Chem. 1985 63, 2787; Mp 75-76°C. Sublimation of such a sample at 100°/0.2mm gave white crystals, Mp. 72-74°C; 'H-NMR (DMSO-d₆) δ: 8.055 (br d,1,NH), 6.68(m,1,=CH), 4.3(m,1,CHN), 3.65(s,3,OMe), 2.9-2.7 and 2.4-2.2 (both m, 4, 2CH₂), 1.75(s,3,MeC=O).

| | | | |
|---|---|---|---|
| Anal. calc. for C₉H₁₃NO₃. 0.1H₂O; | C,58.43; | H,7.19; | N,7.57. |
| Found: | C,58.37; | H,7.34; | N,7.34. |

B. (±)-4-Acetamido-1-cyclopentene methanol
(±)-Methyl-4-acetamido-1-cyclopentene-1-carboxylate (3.66g, 20.0mmol) was dried by evaporation of toluene giving a solution with a final volume of 50mL. This solution was cooled to -70°C under nitrogen. A solution of diisobutylaluminiumhydride in toluene (1.5M, 42mL, 63mmol) was added dropwise over 2 hours. The resulting hazy solution was stirred at -70°C for an additional 40 minutes. Cold methanol (5mL) was added dropwise, followed by a solution of sodium potassium tartrate (11.29g) in water (15mL) with the temperature maintained at -70°C. The resulting mixture was allowed to stir at 0°C for several hours, diluted with methanol (150mL) and filtered. The methanol was evaporated and the residual brown oil (3.44g) chromatographed on silica gel. Title compound was eluted as pale yellow oil with 5% methanol-chloroform; 1.30g (42%); 'H-NMR(DMSO-d₆) δ: 8.00 (d,1,NH), 5.45(m,1,=CH), 4.71(t,J=5.5,1,OH), 4.35-4.2(m,1,CH-N), 4.0-3.9(m,2,CH₂OH), 2.65-2.5 (overlapping DMSO-d₅) and 2.2-2.0 (both m, 4, 2CH₂), 1.77(s,3,CH₃CO); EI-MS: M=155.
C. (±)-9-[3-(Hydroxymethyl)-3-cyclopenten-1-yl)guanine
(±)-4-Acetamido-1-cyclopentene methanol (1.40g, 9.02mmol) was converted by the procedures of Examples 1-4 to (±)-cis-4-(2-amino-6-chloro-9H-purine-9-yl)-1-cyclopentene-1-methanol as a pale yellow solid foam (0.81g, 42%), after elution from a silica gel column with 5-10% methanol-chloroform; 'H-NMR and mass spectrum confirm structure with 5-10% contamination by (±)-cis-4-(2-amino-6-chloro-9H-purin-9-yl)-1-cyclopentyl methanol. Hydrolysis of such a sample (455mg, 1.75mmol) was carried out in dioxane (5mL) and 1N hydrochloric acid (20mL) at 70°C for 12 hours. The resulting solution was brought to pH6 with sodium hydroxide and evaporated to dryness. The residual solids were slurried in methanol and adsorbed on silica gel. Elution with 33% methanol-chloroform gave title compound as a waxy colourless solid. Three crystallisations from water gave white crystals (214mg, 50%); Mp. 260°C; 'H-NMR(DMSO-d₆) δ: 10.54 (s,1,NHCO), 8.14 (s,1,purine H-8), 6.44(s,2,NH₂), 5.70(s,1,=CH), 4.97(m,1,CH-N), 4.85(br t,1,OH), 4.02(s,2,CH₂OH), 2.9-2.7(m,2,CH₂), 2.6-2.5(m, overlapping DMSO-d₅, CH₂).

| | | | |
|---|---|---|---|
| Anal. calc. for C₁₁H₁₃N₅O₂: | C,53.43; | H,5.30; | N,28.32. |
| Found: | C,53.24; | H,5.33; | N,28.27. |

### Example 46

### (±)-cis-4-(2-Amino-6-((2-(dimethylamino)ethyl)amino)-9H-purin-9-yl)-2-cyclopentene-1-methanol

A flask charged with (±)-(cis)-4-(2-amino-6-chloro-9H-purin-9-yl)-2-cyclopentene-1-methanol from Example 4 (399mg, 1.5mmol), N,N-dimethylaminoethylamine (309mg, 35mmol) and ethanol (10mL) was stirred at reflux for 3.5 hours. The solution was cooled to room temperature before the addition of 1.5mL of 1N NaOH. The solution was then concentrated and the residues were placed on a silica gel column. The title compound was eluted with 30% methanol-chloroform then concentrated to yield a colourless glass. Dilution in methanol (5mL) and HCl(0.3mL,12N), followed by evaporation produced a pale yellow solid which was slurried in EtOH; (380mg, 59%); Mp. dec.>185°C 'H-NMR(Me₂SO-d₆) δ 10.4, 9.25, 7.67 (all br, NH⁺, NH₂⁺), 8.06(s,1H, purine H-8), 6.18-6.15 and 5.89-5.86(both m, 2H, HC=CH), 5.45(br s,1H,CH-N) 4.20(br s,1H,H-1'), 3.90(br s,2H,CH₂O), 3.53-3.3(m,4H,NCH₂CH₂N), 2.83 and 2.80 (both s,6H, NMe₂), 2.75-2.55(m,1H, 0.5 CH₂), 1.70-1.50(m,1H, 0.5 CH₂).

| | | | | |
|---|---|---|---|---|
| Anal. calc. for C₁₅H₂₃N₇O.0.3HCl.0.25H₂O: | C,41.77; | H,6.19; | N,22.73; | Cl,24.66. |
| Found: | C,41.51, 41.43; | H,5.88,5.90; | N,22.52,22.48; | Cl,24.61. |

### Example 47

### (±)-cis-Ethyl-2-[(2-Amino-9-(4-(hydroxymethyl)-2-cyclopenten-1-yl)-9H-purin-6-yl)amino]acetate

A solution of (±)-(cis-4-(2-amino-6-chloro-9H-purin-9-yl)-2-cyclopentene-1-methanol from Example 4 (4oomg, 1.50mmol), ethyl glycinate hydrochloride (237mg, 1.70mmol), and triethylamine (516mg, 5.10mmol) in absolute ethanol (10mL) was refluxed under nitrogen for 2 days. The ethanol was evaporated and the residue partitioned between saturated aqueous sodium bicarbonate and chloroform. The chloroform was dried (MgSO₄), evaporated, and the residue chromatographed on silica gel. Title compound was eluted with 5% methanol-chloroform as a pale yellow glass (240mg, 48%) which solidified to off-white powder in acetonitrile ether; Mp. 95-97°C; 'H-NMR (DMSO-d₆) δ: 7.62(s,1, purine H-8), 7.48(br s,1,NH), 6.10(m,1,=CH), 5.84(m,3,=CH and NH₂), 5.37(m,1,CH-N), 4.73(t,J=5.3,1,OH), 4.08(m,4, OCH₂CH₃ and NCH₂), 3.425(t,J=5.5,2,CH₂OH), 2.85(m,1,CH), 2.60 and 1.60(both m, 1 each, CH₂).

| | | | |
|---|---|---|---|
| Anal. calc. for C₁₅H₂₀N₆O₃: | C,54.21; | H,6.07; | N,25.29. |
| Found: | C,54.45; | H,5.93; | N,25.07. |

### Example 48

### (±)-cis-4-(2-Amino-6-piperidino-9H-purin-9-yl)-2-cyclopentene-1-methanol dihydrochloride

A solution of (±)-(cis)-4-(2-amino-6-chloro-9H-purin-9-yl)-2-cyclopentene-1-methanol from Example 4 (399mg, 1.50mmol) and piperidine (298mg, 3.5mmol) in absolute ethanol (10mL) was refluxed under nitrogen for 1 hour. 1N sodium hydroxide (1.5mL) was added and the solution evaporated in dryness. The residue was chromatographed on silica gel. The title compound was eluted with 10% methanol-chloroform as pale yellow glass (0.30g). Precipitated as hydrochloride from acetonitrile; white powder (0.30g, 52%); Mp: dec at 175-180°C 'H-NMR (DMSO-d₆) δ: 7.91 (s,1,purine H-8), 7.70 (br 3,2,NH₂) 6.2-6.1 and 5.9-5.8 (both m, 2,CH=CH), 5.55-5.4 (br m,1,CH-N), 5.4-3.8(br m,NH⁺, 2CH₂N,OH), 3.5-3.35(m, 1,H-1'), 2.75-2.55(m,1, 0.5 CH₂), 1.75-1.50 (m,7,3CH₂ and 0.5 CH₂).

| | | | | |
|---|---|---|---|---|
| Anal. calc. for C₁₆H₂₂N₆O.2HCl: | C,49.62; | H,6.25; | N,21.70; | Cl,18.31. |
| Found: | C,49.54; | H,6.26; | N,21.64; | Cl,18.24. |

### Example 49

### (±)-cis-4-[2-Amino-6(cyclopropylethylamino)-9H-purin-9-yl]-2-cyclopentene-1-methanol

A sample of N-cyclopropyl-N-ethylamine was prepared as follows, cyclopropylamine (18.44g, 0.323mole), potassium carbonate (45.6g, 0.33mole), and dry diethyl ether (250mL) were stirred vigorously with cooling (ice bath) while trifluoroacetic anhydride (50mL, 0.36 mol) was added dropwise over 30 minutes. Ice water (20mL) was added. The ether layer was separated and dried (MgSO₄). Concentration gave a pale yellow liquid (51.1g). A portion of this liquid (15.3g, ca.0.1mole) was dissolved in dry acetone (250mL) with ethyl iodide (46.8g, 0.30 mole) and heated to 70°C (oil bath). Powdered potassium hydroxide (16.8g, 0.300 equiv.) was added. Stirring was continued at 70°C for 30 minutes. Excess ethyl iodide and acetone were removed by evaporation. Water (100mL) was added to the residue and the resulting solution brought to reflux over 15 minutes (oil bath 110°C) and maintained at reflux for 5 minutes. The solution was cooled to 25°C, saturated with sodium chloride, and extracted with diethyl ether (3x100mL). The ether solution was dried (MgSO₄) and evaporated to leave pale yellow oil (4.86g, 57%); 'H-NMR (DMSO-d₆) δ: 7.74 (q,J=6.0,2 NCH₂CH₃), 2.2-2.08 (m,1,CHN), 1.97 (br s,NH + H₂O), 1.19 (t,J=6.0, 3, NCH₂CH₃), 1.9-1.3(m,4,2CH₂). Such a sample of N-cyclopropyl-N-ethylamine (1.26g) was heated with (±)-(cis)-4-(2-amino-6-chloro-9H-purin-9-yl)-2-cyclopentene -1-methanol from Example 4 (544mg, 2.00mmol) in methanol (16mL) in a Parr bomb at 75°C for 11.5 hours. Sodium hydroxide (1N, 1.5mL) was added and the solution evaporated to dryness. The residue was chromatographed on silica gel. Title compound was eluted with 4% methanol-chloroform as pale yellow-glass which crystallised from acetonitrile; 298mg (47%); Mp. 152-154°C; 'H-NMR(DMSO-d₆) δ: 7.64(s,1H, purine H-8), 6.11 and 5.88(m,2,HC=CH), 5.80(br s,2,NH₂), 5.42(m,1,NCH cyclopentene) 4.75(t,J=4.8, 1,OH), 3.94(m,2,NCH₂), 3.45(m,2,OCH₂), 3.05(m,1, NCH cyclopropane), 2.87(br m,1,CH), 2.60(m, overlapping with DMSO, 0.5 CH₂ cyclopentene), 1.56(m,1, 0.5 CH₂ cyclopentene), 1.10(t,J=6.9,CH₃), 0.85 and 0.65(m,4,2CH₂).

| | | | |
|---|---|---|---|
| Anal. calc. for C₁₆H₂₂N₆O: | C,61.13; | H,7.05; | N,26.73. |
| Found: | C,61.06; | H,7.07; | N,26.66. |

### Example 50

### (±)-cis-[4-[2-Amino-6-(cyclopropylamino)-9H-purin-9-yl]-2-cyclopenten-1-yl]methyl L-valinate trifluoroacetate

N-Butyloxycarbonyl-L-valine (1.200g, 5.19mmol) and N,N-dicyclohexylcarbodiimide (0.562g, 2.73mmol) were stirred in dry methylene chloride (46mL) for 40 minutes. The mixture was filtered, the precipitate washed with methylene chloride (8mL), and the filtrate-wash evaporated to dryness. This residual white solid (an hydride) was added in two portions to (±)-cis-4-(2-Amino-6-(cyclopropylamino)-9H-purin-9-yl)-2-cyclopentene-1-methanol from Example 5 (572mg, 2.00mmol), dry N,N-dimethylformamide (19mL) and 4-N,N-dimethylaminopyridine (20mg, 0.16mmol). The reaction was stirred at 7.5°C under nitrogen for 69 hours, before the addition of water (0.3mL). The solution was evaporated to dryness and the residue partitioned between chloroform and NaHCO₃ (2.5mmol) in H₂O. The aqueous layer was extracted with chloroform and the combined organic layers were dried (MgSO₄), filtered. Elution with 4% methanol-chloroform gave the N-butyloxy-carbonyl-blocked derivative of the title compound as white foam (520g); 'H-NMR(Me₂SO-d₆) δ: 7.62 (s,1, purine H-8), 7.30(d,J=3.9, 1, NH cyclopropylamine), 7.16(d,J=7.9,1,CHN), 6.08 and 5.95 (m,2,HC=CH), 5.83(br s, 2, NH₂), 5.42(br m, 1, NCH), 4.13(d,J=6.3,2, OCH₂), 3.82(t,J=7.4,1, NCH of valyl), 3.08(br m,2, CH cyclopentene, CH cyclopropane) 2.69(m,1, 0.5 CH₂ cyclopentene), 1.97(m,1, CHMe₂), 1.60(m,1, 0.5 CH₂ cyclopentene), 1.37(s,9, C(CH)₃), 0.88-0.79 (overlapping d,6, CH(CH₃)₂), 0.64 and 0.59(m,4, 2CH₂ cyclopropane). This derivative (510mg) was dissolved in trifluoracetic acid: methylene chloride/1:3 (∼25mL) and the solution stirred at 25°C for 30 minutes. Evaporation left the title compound as a yellow foam (745mg); 'H-NMR (DMSO-d₆): δ 9.85 (br m, 1,NH), 8.37(br m, 3, NH₃⁺), 8.01(br s, 1, purine H-8), 7.57(br s, 2, NH₂), 6.17 and 6.02(m,2, HC=CH), 5.48(m,1,NCH cyclopentene), 4.26(m, overlapping br solvent, OCH₂), 3.94(br m, overlapping solvent, valyl CH), 3.17(m,1,CH), 2.9-2.68(br m,2, cyclopropyl CHN, 0.5 CH₂ cyclopentene), 2.14(m,1, CHMe₂), 1.66(m,1, 0.5 CH₂ cyclopentene), 0.94(m,8, CHMe₂, CH₂ cyclopropane), 0.78(m,2,cyclopropyl CH₂).

| | | | |
|---|---|---|---|
| Anal. calc. for C₁₉H₂₇N₇O₂.0.8H₂O.3.8 CF₃CO₂H: | C,38.35; | H,3.92; | N,11.77. |
| Found: | C,38.25; | H,3.79; | N,11.80. |

### Example 51

### (±)-cis-4-[2-Amino-6(cyclobutylmethylamino)-9H-purin-9-yl]-2-cyclopentene-1-methanol

A sample of N-cyclobutyl-N-methylamine was prepared as follows. Cyclobutylamine (5.00g, 68.9mmol) and potassium carbonate (13.3g, 96.5mmol) were vigorously stirred in dry diethyl ether (250mL) under nitrogen in an ice bath while trifluoroacetic anhydride (10.7mL) was added dropwise over 20 minutes. Ice water (20mL) was added. The ether layer was separated, dried (MgSO₄), and concentrated to a colourless liquid (11.50g). This liquid was dissolved in dry acetone (170mL) with methyl iodide (40g, 0.28mole) and heated to 40°C. Powdered potassium hydroxide (16g, 0.28 equiv.) was added. Stirring at 40°C was continued for 45 minutes. The excess methyl iodide and acetone were removed by evaporation and water (75mL) was added to the residual liquid and solids. The resulting solution was brought to reflux over 15 minutes (oil bath 110°C) and maintained at reflux for 5 minutes. The solution was cooled to 25°C, saturated with sodium chloride, and extracted with diethyl ether (3x150mL). The ether solution was dried (MgSO₄) and evaporated to leave colourless oil (3.72g, 64%); 'H-NMR (CDCl₃) δ: 3.25-3.15(m,1, CHN), 2.34(s,3, NCH₃), 2.27-2.03(m,2, 2CH), 1.8-1.6(m,4, CH₂ and 2CH). Such a sample of N-cyclobutyl-N-methylamine (510mg, 6.0mmol) was heated with (±)-(cis)-4-(2-amino-6-chloro-9H-purin-9-yl)-2-cyclopentene-1-methanol from Example 4 (544mg, 2.00mmol) in methanol (16mL) in a Parr bomb at 65°C for 5.5 hours. Sodium hydroxide (1N, 2mL) was added and the solution evaporated to dryness. The residue was chromatographed on silica gel. Title compound was eluted with 10% methanol-chloroform as a pale yellow solid foam, from aceonitrile; 528mg, 84%; 'H-NMR (DMSO-d₆) δ: 7.62(s,1, purine H-8), 6.15-6.07(m,1,=CH), 6.0-5.7(m,4,=CH, NH₂, and cyclobutyl CHN), 5.5-5.3(m,1,CHN), 4.77(t,J=5.3,1,OH), 3.42(m,2, CH₂OH), 3.27(s, overlapped by H₂O, N-CH₃), 2.85(m,1,H-1'), 2.7-2.5(m,1, 0.5 cyclopentyl CH₂), 2.4-2.0(m 4, 2 cyclobutyl CH₂), 1.7-1.4(m,3, 0.5 cyclopentyl CH₂ and cyclobutyl CH₂).

| | | | |
|---|---|---|---|
| Anal. calc. for C₁₆H₂₂N₆O.03H₂O.0.05 CH₃CH: | C,60.08; | H,7.12; | N,26.33 |
| Found: | C,60.02,59.97; | H,7.10,7.13; | N,26.30,26.26. |

### Example 52

### (±)-cis-[4-[2-Amino-6-(cyclopropylmethylamino)-9H-purin-9-yl]-2-cyclopentene-1-yl)-methyl-L-valinate trifluoroacetate

N-Butyloxycarbonyl-L-valine(1.09g, 5.0mmol) and N,N-dicyclohexylcarbodiimide (0.515g, 2.5mmol) were stirred in dry methylenechloride (15mL) for 1 hour. The mixture was filtered, the precipitate was washed with methylene chloride (10mL), and the filtrate-wash was evaporated to dryness. To this was added (±)-cis-4-(2-amino-6-(cyclopropylmethylamino)-9H-purin-9-yl]-2-cyclopentene-1-methanol from Example 29 (0.600g, 2mmol), dry N,N-dimethylformamide (15mL) and N,N-dimethylaminopyridine (5mg, 0.04mmol). The reaction was stirred at room temperature under nitrogen for 16 hours. H₂O (1mL) was added and the solution was concentrated under vacuum. The residual oil was partitioned between 0.1N NaOH (2mL) and chloroform(3x50mL). The combined chloroform extracts were dried (MgSO₄), solvent evaporated and the residue chromatographed on silica gel. Elution with 5% methanol-chloroform gave the N-butyloxycarbonyl-blocked derivative of the title compound as a white solid (0.750g, 75%); 'H-NMR (DMSO-d₆) δ: 7.62(s,1, H-8), 7.15(d,J=8.2,1,NH), 6.10 and 5.90 (2m,2,CH=CH), 5.79(br s,2,NH₂), 5.40(br m,1,CH-N), 4.10(d,J=6.4,2,CH₂-O), 3.80(m,1 valyl CH-N), 3.30-3.15(m, overlapping s at 3.23, total 4, CH-N-Me), 3.10-3.0(br m,1,CH), 2.75-2.55(m,1, 0.5 CH₂), 2.05-1.85(m,1,CHMe₂), 1.70-1.50(m,1, 0.5 CH₂), 1.34(m,9,CMe₃), 0.90-0.60(m,10,CHMe₂ and cyclopropyl CH₂). Such a sample (0.74g, 1.5mmol) was dissolved in trifluoroacetic acid: methylene chloride/1:3 (25mL) and the solution stirred at 25°C under nitrogen for 30 minutes. Evaporation left the title compound as a yellow foam (0.957g, 87%) 'H-NMR (DMSO-d₆) δ: 8.38(br s,3,NH₃⁺), 8.0(s,1,H-8), 7.80-7.10(br m,2, NH₂), 6.18 and 6.0(2m,2,CH=CH), 5.48(m,1,CH-N), 4.26(br d,J=6.56, overlapped by H₂O, CH₂-O), 3.93(br m,1, CH-N valyl), 3.55(br s,3, N-Me), 3.20-3.10(br m,2,CH and cyclopropyl CH-N), 2.79-2.69(m,1, 0.5 CH₂), 2.20-2.05(m,1,CHMe₂), 1.67-1.60(m,1, 0.5 CH₂), 1.10-0.90(m,10,2 CH₃ and 2 cyclopropyl CH₂).

| | | | |
|---|---|---|---|
| Anal. calc. for C₂₀H₂₉N₇O₂.1.0H₂O.0.4 EtOH.2.60 CF₃ CO₂H: | C,42.64; | H,4.95; | N,13.39. |
| Found: | C,42.63; | H,4.91; | N,13.42 |

### Example 53

### (±)-cis-4-(2-Amino-6-(cyclobutylthio)-9H-purin-9-yl)-2-cyclopentene-1-methanol

A mixture of (±)-cis-2-amino-1,9-dihydro-9-[(4-hydroxymethyl)-2-cyclopenten-1-yl]-6H-purine-6-thione hydrochloride (500mg, 1.45mmol), potassium carbonate (600mg) and cyclobutylbromide (0.98g, 7.25mmol), added in 5 portions over 18 hours in dry N,N-dimethylformamide (20mL) was stirred under nitrogen for 24 hours at 25°C. The N,N-dimethylformamide was removed under reduced pressure. The residual oil was partitioned between chloroform and water. The chloroform layer was dried (MgSO₄) and concentrated to a yellow glass which was chromatographed on silica gel. Title compound was eluted with 6% methanol-chloroform and crystallised four times from acetonitrile to give pale yellow granules (0.115g, 25%) Mp. 159-160°C; ,H-NMR (DMSO-d₆) δ: 7.81(s,1,H-8), 6.41(br s,2,NH₂), 6.10 and 5.85(2 m, 2,CH=CH), 5.40(br m,1,CH-N), 4.69(t,J=5.3) overlapping 4.70-4.50(m, total 2, OH and S-CH), 3.45(m,1,CH₂-O), 2.80(br m,1,CH), 2.70-1.90(m, overlapping solvent, 0.5 CH₂ and 3 cyclobutyl CH₂), 1.70-1.50(m,1, 0.5 CH₂).

| | | | | |
|---|---|---|---|---|
| Anal. calc. for C₁₅H₁₉N₅OS: | C,56.76; | H,6.03; | N,22.06; | S,10.10. |
| Found: | C, 56.75; | H, 6.07; | N, 21.98; | S, 10.04. |

### Example 54

### (±)-cis-4-(2-Amino-6-((2,3-dihydroxypropvl)amino)-9H-purin-9-yl)-2-cyclopentene-1-methanol

A solution of (±)-cis-4-(2-amino-6-chloro-9H-purin-9-yl)-2-cyclopentene -1-methanol from Example 4 (0.544, 2mmol), 3-amino-1,2-propanediol (1.87mg, 2mmol), triethylamine (607mg, 6mmol) and methoxyethanol (6ml) were refluxed overnight under nitrogen. 1NNaOH (2ml) was added and the solution was concentrated under vacuum and dried by evaporation of ethanol. The residual oil was chromatographed on silica gel. Title compound was eluted with 20% methanol-chloroform; white powder after crystallisation from acetonitrile-methanol (0.300g, 47%), Mp. 119-121°C; 'H-NMR (DMSO-d₆)δ; 7.60 (s, 1, H-8), 6.90-6.80 (br m,1,NH), 6.10 (m,1,=CH), 5.85 (m,3,=CH and NH₂), 5.40 (br m,1,CH-N), 4.90 (m,1,OH), 4.72 (t,J=5.3,1,OH), 4.62 (t,J=5.9,1,OH), 3.7-3.25 (all m, overlapping H₂O, 2CH₂-O and CH-O), 2.85 (br m,1,CH), 2.70-2.50 (m, overlapping solvent, 0.5 CH₂), 1.70-1.50 (m,1,0/5 CH₂).

| | | | |
|---|---|---|---|
| Anal. calc. C₁₄H₂₀N₆O₃0.5H₂O: | C, 51.06; | H, 6.43; | N 25.52. |
| Found: | C, 50.99, 50.96; | H, 6.45, 6.49; | N 25.42, 25.36. |

### Example 55

### (±)-cis-4-[2-Amino-6-(cyclopropylamino)-8-methyl-9H-purin-9-yl]-2-cyclopentene-1-methanol

(±)-cis-4-[(2,5-Diamino-4-chloro-6-pyrimidinyl)amino]-2-cyclopentene-1-methanol prepared as in Example 3 (1.12g, 4.38mmol) was stirred in N,N-dimethylformamide (5mL) with trimethylorthoacetate (30mL) and ethane sulfonic acid (0.66g, 5.7mmol) at 70°C for 3 days. The resulting solution was evaporated to a yellow syrup. Acetic anhydride (20mL) was added and this solution was refluxed for 2.5 hours. The resulting dark solution was evaporated to a syrup, which was dissolved in 1N hydrochloric acid (50mL). After 24 hours, the pH was adjusted to 6 with sodium hydroxide and most of the water evaporated. Crude product was extracted into 20% isopropyl alcohol-chloroform. This solution was dried (MgSO₄) and solvent evaporated to leave (±)-cis-4-(2-amino-6-chloro-8-methyl-9H-purin-9-yl)-2-cyclopentene-1-methanol as a pale yellow glass (0.30g); structure confirmed by 'H-NMR. This sample was dissolved in methanol (10mL) and stirred in a Parr bomb with cyclopropylamine (1mL) at 70°C for 12 hours. Evaporation and chromatrography on silica gel gave title compound, eluted as a cream-colored solid foam (136mg) with 5% methanol-chloroform; 'H-NMR (DMSO-d₆)δ: 7.13 (d,J=4.6,1,NH), 6.02 and 5.84 (both m, 2,CH=CH), 5.68-5.56 (m,3,NH₂ and CH-N), 4.85 (t,1,CH₂OH), 3.53 (m,2,CH₂OH), 3.02 (m,1,CH-N of cyclopropyl), 2.88 (m,1,CH), 2.5 (m, overlapping solvent, 0.5 cyclopentyl CH₂), 1.72 (m,1, 0.5 cyclopentyl CH₂), 0.7-0.5 (m,4,2 cyclopropyl CH₂).

| | | | |
|---|---|---|---|
| Anal. calc. for C₁₅H₂₀N₆O.0.25CH₃OH·065H₂O: | C,57.23; | H,7.02; | N,26.26. |
| Found | C,57.55; | H,6.99; | N,25.95. |

### Example 56

### (±)-cis-4-(2-Amino-6-((2-hydroxy-1-(hydroxymethyl)ethyl)amino)-9H-purin-9-yl)-2-cyclopentene-1-methanol

A solution of serinol hydrochloride (0.765g, 6.00mmol) in methanol (20mL) was stirred with basic ion exchange resin for 10 minutes. The resin was filtered off and the filtrate concentrated to a colorless oil. To this was added (±)-cis-4-(2-amino-6-chloro-9H-purin-9-yl)-2-cyclopentene-1-methanol (0.544g, 2.00mmol) from Example 4 and methanol (10mL). The resulting solution was stirred in a Parr bomb at 80°C overnight. 1N NaOH (2mL) was added and the solvent evaporated.

The residue was chromatrographed on silica gel. Title compound eluted with 20% methanol-chloroform; white powder after crystallization from acetonitrile-methanol (0.404g, 63%), Mp. 160-162°C; 'H-NMR (DMSO-d₆)δ: 7.60 (s,1,H-8), 6.38 (m,1,NH), 6.10(m,1,=CH), 5.90-5.75 (m, overlapped by s at 5.8; total 3, =CH and NH₂), 5.40 (br m,1,CH-N), 4.70 (m,3,30H), 4.20 (br m,1,CH-NH), 3.60-3.40 (2 m,6,3 CH₂-O), 2.75 (br m,1,CH), 2.70-2.50 (m,1, 0.5 CH₂), 1.65-1.50 (m,1, 0.5 CH₂).

| | | | |
|---|---|---|---|
| Anal. calc. for C₁₄H₂₀N₆O₃: | C,52.49; | H,6.29; | N,26.24. |
| Found: | C,52.38; | H,6.33; | N,26.23. |

### Example A

### Tablet Formulations

The following formulations A, B and C are prepared by wet granulation of the ingredients with a solution of povidone, followed by addition of magnesium stearate and compression.

### Formulation A

| | mg/tablet | mg/tablet |
|---|---|---|
| (a) Active ingredient | 250 | 250 |
| (b) Lactose B.P. | 210 | 26 |
| (c) Povidone B.P. | 15 | 9 |
| (d) Sodium Starch Glycollate | 20 | 12 |
| (e) Magnesium Stearate | 5 | 3 |
| | 5̅0̅0̅ | 3̅0̅0̅ |

### Formulation B

| | mg/tablet | mg/tablet |
|---|---|---|
| (a) Active ingredient | 250 | 250 |
| (b) Lactose | 150 | - |
| (c) Avicel PH 101 | 60 | 26 |
| (d) Povidone B.P. | 15 | 9 |
| (e) Sodium Starch Glycollate | 20 | 12 |
| (f) Magnesium Stearate | 5 | 3 |
| | 5̅0̅0̅ | 3̅0̅0̅ |

### Formulation C

| | mg/tablet |
|---|---|
| Active ingredient | 100 |
| Lactose | 200 |
| Starch | 50 |
| Povidone | 5 |
| Magnesium stearate | 4 |
| | 3̅5̅9̅ |

The following formulations, D and E, are prepared by direct compression of the admixed ingredients. The lactose in formulation E is of the direct compression type (Dairy Crest - "Zeparox").

### Formulation D

| | mg/tablet |
|---|---|
| Active ingredient | 250 |
| Pregelatinised Starch NF15 | 150 |
| | 4̅0̅0̅ |

### Formulation E

| | mg/tablet |
|---|---|
| Active ingredient | 250 |
| Lactose | 150 |
| Avicel | 100 |
| | 5̅0̅0̅ |

### Formulation F (Controlled Release Formulation)

The formulation is prepared by wet granulation of the ingredients (below) with a solution of povidone followed by the addition of magnesium stearate and compression.

| | mg/tablet |
|---|---|
| (a) Active ingredient | 500 |
| (b) Hydroxypropylmethylcellulose (Methocel K4M Premium) | 112 |
| (c) Lactose B.P. | 53 |
| (d) Povidone B.P. | 28 |
| (e) Magnesium Stearate | 7 |
| | 7̅0̅0̅ |

Drug release takes place over a period of about 6-8 hours and is complete after 12 hours.

### Example B

### Capsule Formulations

### Formulation A

A capsule formulation is prepared by admixing the ingredients of Formulation D in Example A above and filling into a two-part hard gelatin capsule. Formulation B (infra) is prepared in a similar manner.

### Formulation B

| | mg/capsule |
|---|---|
| (a) Active ingredient | 250 |
| (b) Lactose B.P. | 143 |
| (c) Sodium Starch Glycollate | 25 |
| (d) Magnesium Stearate | 2 |
| | 4̅2̅0̅ |

### Formulation C

| | mg/capsule |
|---|---|
| (a) Active ingredient | 250 |
| (b) Macrogol 4000 B.P. | 350 |
| | 6̅0̅0̅ |

Capsules of formulation C are prepared by melting the Macrogol 4000 BP, dispersing the active ingredient in the melt and filling the melt into a two-part hard gelatin capsule.

### Formulation D

| | mg/capsule |
|---|---|
| Active ingredient | 250 |
| Lecithin | 100 |
| Arachis Oil | 100 |
| | 4̅5̅0̅ |

Capsules of formulation D are prepared by dispersing the active ingredient in the lecithin and arachis oil and filling the dispersion into soft, elastic gelatin capsules.

### Formulation E (Controlled Release Capsule)

The following controlled release capsule formulation is prepared by extruding ingredients a, b and c using an extruder, followed by spheronisation of the extrudate and drying. The dried pellets are then coated with release- controlling membrane (d) and filled into a two-piece, hard gelatin capsule.

| | mg/capsule |
|---|---|
| (a) Active ingredient | 250 |
| (b) Microcrystalline Cellulose | 125 |
| (c) Lactose B.P. | 125 |
| (d) Ethyl Cellulose | 13 |
| | 5̅1̅3̅ |

### Example C

### Injectable Formulation

### Formulation A.

| | |
|---|---|
| Active ingredient | 0.200g |
| Hydrochloric acid solution, 0.1M, or Sodium hydroxide solution, 0.1M q.s. to pH | 4.0 to 7.0 |
| Sterile water q.s. to | 10ml |

The active ingredient is dissolved in most of the water (35°-40°C) and the pH adjusted to between 4.0 and 7.0 with the hydrochloric acid or the sodium hydroxide as appropriate. The batch is then made up to volume with the water and filtered through a sterile micropore filter into a sterile 10ml amber glass vial (type 1) and sealed with sterile closures and overseals.

### Formulation B.

| | |
|---|---|
| Active ingredient | 0.125 g |
| Sterile, pyrogen-free, pH 7 phosphate buffer, q.s. to | 25 ml |

### Example D

### Intramuscular injection

| | |
|---|---|
| Active ingredient | 0.20 g |
| Benzyl Alcohol | 0.10 g |
| Glycofurol 75 | 1.45 g |
| Water for Injection q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml amber glass vials (type 1).

### Example E

### Syrup

| | |
|---|---|
| Active ingredient | 0.25 g |
| Sorbitol Solution | 1.50 g |
| Glycerol | 2.00 g |
| Sodium Benzoate | 0.005 g |
| Flavour, Peach 17.42.3169 | 0.0125 ml |
| Purified Water q.s. to | 5.00 ml |

The active ingredient is dissolved in a mixture of the glycerol and most of the purified water. An aqueous solution of the sodium benzoate is then added to the solution, followed by addition of the sorbitol solution and finally the flavour. The volume is made up with purified water and mixed well.

### Example F

### Suppository

| | mg/suppository |
|---|---|
| Active ingredient (63»m)* | 250 |
| Hard Fat, BP (Witepsol H15 - Dynamit NoBel) | 1770 |
| | 2̅0̅2̅0̅ |

| | |
|---|---|
| *The active ingredient is used as a powder wherein at least 90% of the particles are of 63»m diameter or less. | |

One-fifth of the Witepsol H15 is melted in a steam-jacketed pan at 45°C maximum. The active ingredient is sifted through a 200»m sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250»m stainless steel screen and, with continuous stirring, is allowed to cool to 40°C. At a temperature of 38°C to 40°C, 2.02g of the mixture is filled into suitable, 2 ml plastic moulds. The suppositories are allowed to cool to room temperature.

### Example G

### Pessaries

| | mg/pessary |
|---|---|
| Active ingredient (63»m) | 250 |
| Anhydrate Dextrose | 380 |
| Potato Starch | 363 |
| Magnesium Stearate | 7 |
| | 1̅0̅0̅0̅ |

The above ingredients are mixed directly and pessaries prepared by direct compression of the resulting mixture.

### Antiviral Activity

The compounds of Examples 5 and 6 were tested for anti-HIV activity in MT₄ cells generally in accordance with the method described by Mitsuya et al, Proc.Nat.Acad.Sci; USA Vol 82, pp 7096-7100, October 1985, and were found to have IC₅₀ values of 32.7 »M and 13.7 »M, respectively. A repeat determination with the compound of Example 5 gave 11 »M.

Determination of anti-HBV activity is carried out by testing the ability of a compound to prevent replication of duck HBV in vitro, in the manner described by Tuttleman, Pugh and Summers (Journal of Virology, 58:17-25, 1986). Duck hepatocytes are obtained and placed in culture, and infected with duck HBV. Three days after infection, the infected cells are exposed to various concentrations of the test compound for an additional period of eight days. After this exposure, DNA is extracted from each culture of infected cells and compound, and the amount of viral DNA is specifically determined and compared with that obtained from similar cultures lacking the test compound.

### Toxicity Data

### Determination of Growth Inhibition of Uninfected Mammalian Cells

The capability of candidate compounds to inhibit the growth of D98 cells (human) and L cells (murine) was measured by determination of cell number following three days exposure of a standard number of cells to various dilutions of compound (Rideout, J.L., Krenitsky, T.A., Koszalka, G.W., Cohn, N.K., Chao, E.Y. Elion, G.B., Latter, V.S., and Williams, R.B. (1982) J. Med Chem. 25: 1040-1044). The cell number was then compared to the number obtained in the absence of compound. Cell enumeration was performed by either direct particle counts following trypsinization of the monolayer, or by spectrophotometric determination of the amount of vital stain taken up by the cells. Comparable results were obtained with both methods.

### Data Analysis

The concentration of compound resulting in 50% of control values (IC50) was calculated either by direct interpolation from graphs of the log of the compound concentration versus the percent of control value, or from a computer program which analyses the data according to the same algorithm. Data in the range of 20% to 80% of control were used in these calculations.

All compounds were tested in D-98 cells and found to have an IC₅₀ value of >100»m.

## Claims (Claims for the following Contracting State(s): AT, BE, CH, DE, FR, GB, IT, LI, LU, NL, SE)

1. A compound of formula (I): 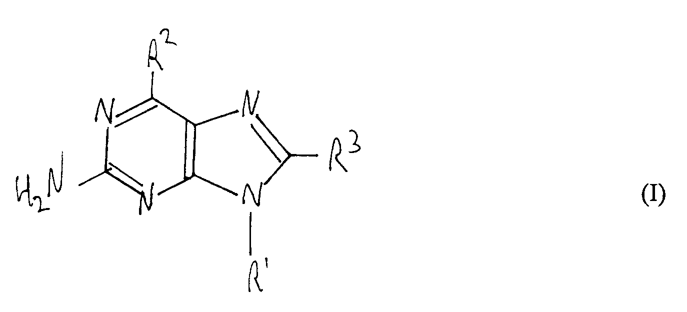 wherein R¹ represents 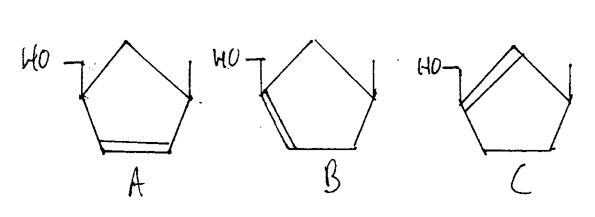 and R² represents branched or straight chain C₁₋₆ alkoxy, optionally substituted by C₁₋₆ alkoxy, C₃₋₆ cycloalkyl; C₃₋₆ cycloalkyl; C₃₋₈ cycloalkyloxy; aryloxy; aralkyl or aralkyloxy in which the aryl may optionally be substituted with C₁₋₄ alkyl, hydroxy or halogen; alkenylthio; C₃₋₆ cycloalkylthio; C₁₋₆ alkylthio; arylthio or aralkylthio in which the aryl may optionally be substituted with C₁₋₄ alkyl, hydroxy, halogen or nitro; or R² represents a heterocyclic group containing an oxygen atom or one or two nitrogen atoms, and 3-7 carbon atoms with optional double bonds in the ring and optionally containing one or more heteroatoms selected from sulphur and oxygen and optionally substituted on the ring by one or more C₁₋₄ alkyl, hydroxy or halogen groups, C₃₋₆ cycloalkylthio, aralkylthio in which the aryl may be substituted with C₁₋₄ alkyl, hydroxy or halogen; or R² represents an imidazolylthio group in which the imidazolyl moiety may be substituted with one or more substituents selected from C₁₋₄ alkyl and C-substituted with nitro; or R² represents an amino group which is mono- or di-substituted by one or two substituents selected from C₁₋₆ alkyl, C₁₋₆ alkoxy, C₁₋₆ hydroxyalkyl, C₃₋₇ cycloalkyl optionally substituted by C₁₋₆ alkyl, aryl, aralkyl in which the aryl may optionally be substituted with C₁₋₄ alkyl, hydroxy or halogen, allyl optionally substituted with mono- or dialkyl or alkoxy groups; and R³ represents hydrogen, amino or C₁₋₆ alkyl; or a pharmaceutically acceptable derivative thereof.

2. A compound of formula (I) according to Claim 1 wherein R¹ represents A

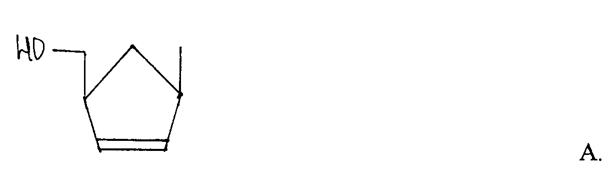

3. A compound according to Claim 1 of formula : 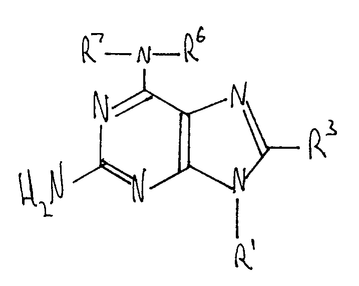 wherein R¹ represents 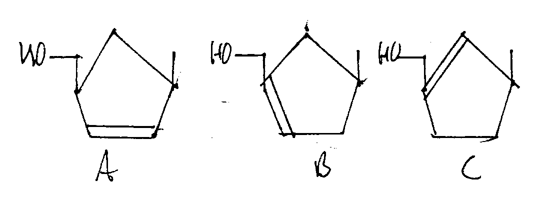 R³ represents hydrogen, amino or C₁₋₆ alkyl; R⁶ represents C₃₋₆ cycloalkyl; and R⁷ represents a hydrogen atom or a substituent selected from C₁₋₆ alkyl, C₁₋₆ alkoxy, C₁₋₆ hydroxyalkyl, C₃₋₆ cycloalkyl optionally substitued by C₁₋₆ alkyl, aryl, aralkyl in which the aryl may optionally be substituted with C₁₋₄ alkyl, hydroxy or halogen, allyl optionally substituted with mono- or di-alkyl or alkoxy groups; or a pharmaceutically acceptable derivative thereof.

4. (±)-cis-4-[2-Amino-(6-cyclopropylamino)-9H-purin-9-yl]-2-cyclopentene-1-methanol.

5. A pharmaceutically acceptable derivative of the compound (±)-cis-4-[2-Amino-(6-cyclopropylamino)-9H-purin-9-yl]-2-cyclopentene-1-methanol.

6. A pharmaceutically acceptable salt of the compound (±)-cis-4-[2-Amino-(6-cyclopropylamino)-9H-purin-9-yl]-2-cyclopentene-1-methanol.

7. A compound according to any of Claims 1 to 5 or a pharmaceutically acceptable derivative thereof for use in medical therapy.

8. A compound according to any of Claims 1 to 5 for use in the treatment or prophylaxis of HIV infections.

9. A compound according to any of Claims 1 to 5 for use in the treatment or prophylaxis of HBV infections.

10. A compound according to any of Claims 1 to 5 for use in the preparation of a medicament for the treatment or prophylaxis of retroviral infections or HBV infections.

11. A process for the preparation of a compound of formula (I) as defined in Claim 1 comprising:
A) treating a compound of formula (II) 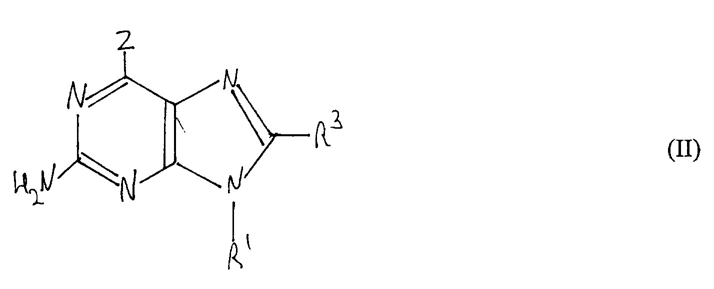 wherein R¹ represents 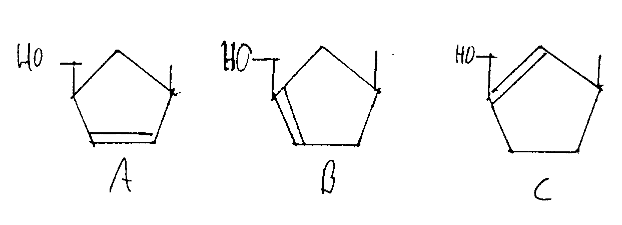 wherein R¹ and R³ are as hereinbefore defined and Z represents a precursor group for the said R² group with an agent or under conditions serving to convert the precursor Z group to the desired R² group; or Z represents a thio group onto which may be substituted an appropriate group to form a compound of formula (I) wherein R² is a substituted thio group; or
B) reacting a compound of formula (III) 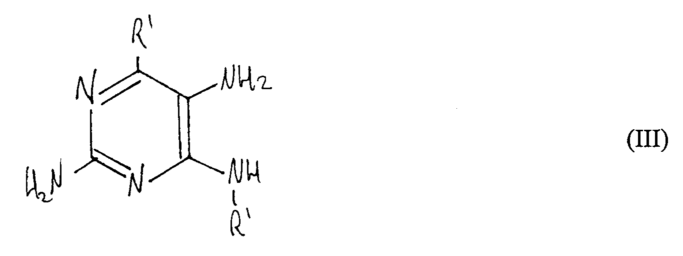 (wherein R¹ and R² are hereinbefore defined) or a pharmaceutically acceptable derivative thereof, with an agent serving to effect formation of the imidazole ring in the desired compound of formula (I); or
i) where a compound of formula (I) is formed, converting the said compound to a pharmaceutically acceptable derivative thereof; or
ii) where a pharmaceutically acceptable derivative of a compound of formula (I) is formed, converting the said derivative to the parent compound of formula (I) or to a further such derivative.

12. A pharmaceutical formulation comprising as active ingredient a compound of formula (I) as defined in any of Claims 1 to 5 and a pharmaceutically acceptable carrier thereof.

13. A pharmaceutical formulation according to Claim 11 in which the pharmaceutical formulation is formed into a tablet or a capsule.

## Claims (Claims for the following Contracting State(s): ES, GR)

1. A process for the preparation of a compound of formula (I) 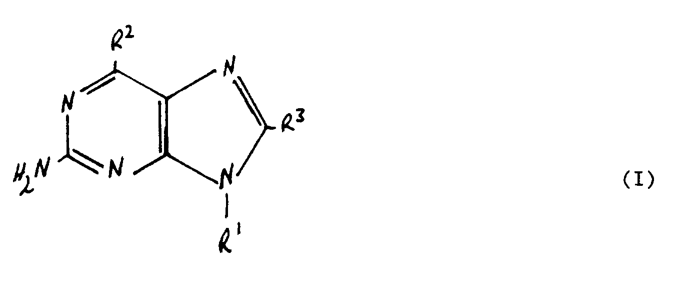 wherein R¹ represents 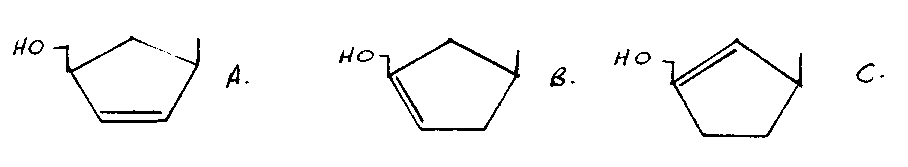 and R² represents branched or straight chain C₁₋₆ alkoxy, optionally substituted by C₁₋₆ alkoxy, C₃₋₆ cycloalkyl; C₃₋₆ cycloalkyl; C₃₋₈ cycloalkyloxy; aryloxy; aralkyl or aralkyloxy in which the aryl may optionally be substituted with C₁₋₄ alkyl, hydroxy or halogen; C₁₋₆ alkenylthio; C₃₋₆ cycloalkylthio; C₁₋₆ alkylthio; arylthio or aralkylthio in which the aryl may optionally be substituted with C₁₋₄ alkyl, hydroxy, halogen or nitro; or R² represents a heterocyclic group containing an oxygen atom or one or two nitrogen atoms, and 3-7 carbon atoms with optional double bonds in the ring and optionally containing one or more heteroatoms selected from sulphur and oxygen and optionally substituted on the ring by one or more C₁₋₄ alkyl, hydroxy or halogen groups, C₃₋₆ cycloalkylthio, aralkylthio in which the aryl may be substituted with C₁₋₄ alkyl, hydroxy or halogen; or R² represents an imidazolylthio group in which the imidazolyl moiety may be substituted with one or more substituents selected from C₁₋₄ alkyl and C-substituted with nitro; or R² represents an amino group which is mono- or di- substituted by one or two substituents selected from C₁₋₆ alkyl, C₁₋₆ alkoxy, C₁₋₆ hydroxyalkyl, C₃₋₇ cycloalkyl optionally substituted by C₁₋₆ alkyl, aryl, aralkyl in which the aryl may optionally be substituted with C₁₋₄ alkyl, hydroxy or halogen, allyl optionally substituted with mono- or di-alkyl or alkoxy groups; and R³ represents hydrogen, amino or C₁₋₆ alkyl; or a pharmaceutically acceptable derivatives thereof,
comprising:
A) treating a compound of formula (II) 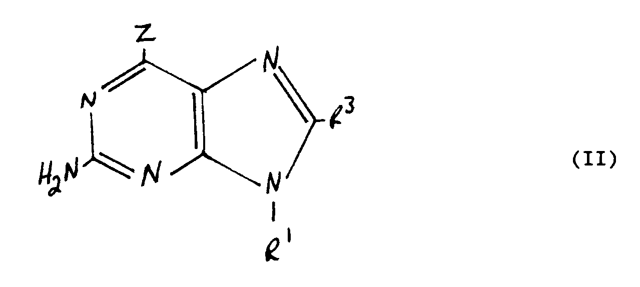 wherein R¹ and R³ are as hereinbefore defined and Z represents a precursor group for the said R² group with an agent or under conditions serving to convert the precursor Z group to the desired R² group; or Z represents a thio group onto which may be substituted an appropriate group to form a compound of formula (I) wherein R² is a substituted thio group; or
B) reacting a compound of formula (III) 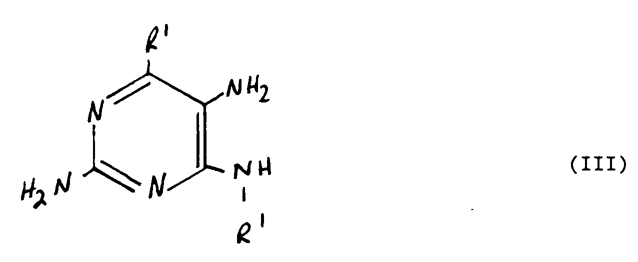 (wherein R¹ and R² are hereinbefore defined) or a pharmaceutically acceptable derivative thereof, with an agent serving to effect formation of the imidazole ring in the desired compound of formula (I); or
i) where a compound of formula (I) is formed, converting the said compound to a pharmaceutically acceptable derivative thereof; or
ii) where a pharmaceutically acceptable derivative of a compound of formula (I) is formed, converting the said derivative to the parent compound of formula (I) or to a further such derivative.

2. A process according to claim 1 for the preparation of a compound of formula (I) wherein R¹ represents

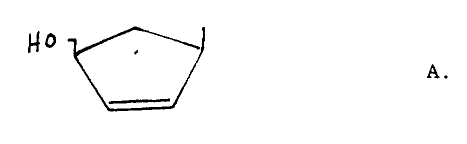

3. A process according to claims 1 or 2 for the preparation of a compound of formula : 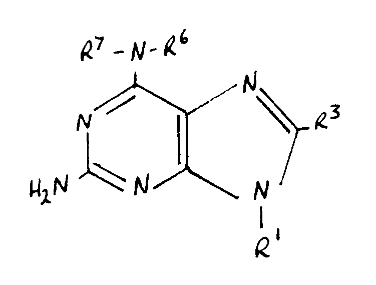 wherein R¹ represents 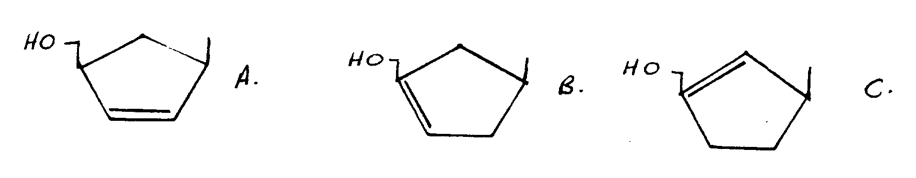 R³ represents hydrogen, amino or C₁₋₆ alkyl; R⁶ represents C₃₋₆ cycloalkyl; and R⁷ represents a hydrogen atom or a substituent selected from C₁₋₆ alkyl, C₁₋₆ alkoxy, C₁₋₆ hydroxyalkyl, C₃₋₆ cycloalkyl optionally substituted by C₁₋₆ alkyl, aryl, aralkyl in which the aryl may optionally be substituted with C₁₋₄ alkyl, hydroxy or halogen, allyl optionally substituted with mono- or di-alkyl or alkoxy groups; or a pharmaceutically acceptable derivative thereof.

4. A process according to claim 1 for the preparation of (±)-cis-4-[2-amino-(6-cyclopropylamino)-9H-purin-9-yl]-2-cyclopentene-1-methanol.

5. A process for the preparation of a pharmaceutical formulation containing a compound of formula (I) 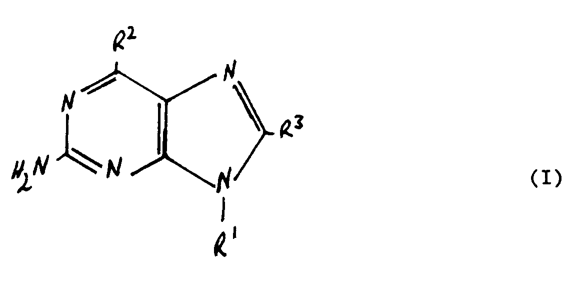 wherein R¹ represents 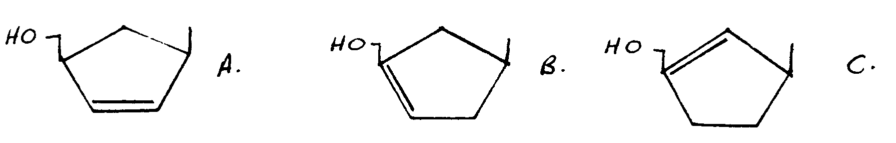 and R² represents branched or straight chain C₁₋₆ alkoxy, optionally substituted by C₁₋₆ alkoxy, C₃₋₆ cycloalkyl; C₃₋₆ cycloalkyl; C₃₋₈ cycloalkyloxy; aryloxy; aralkyl or aralkyloxy in which the aryl may optionally be substituted with C₁₋₄ alkyl, hydroxy or halogen; C₁₋₆ alkenylthio; C₃₋₆ cycloalkylthio; C₁₋₆ alkylthio; arylthio or aralkylthio in which the aryl may optionally be substituted with C₁₋₄ alkyl, hydroxy, halogen or nitro; or R² represents a heterocyclic group containing an oxygen atom or one or two nitrogen atoms, and 3-7 carbon atoms with optional double bonds in the ring and optionally containing one or more heteroatoms selected from sulphur and oxygen and optionally substituted on the ring by one or more C₁₋₄ alkyl, hydroxy or halogen groups, C₃₋₆ cycloalkylthio, aralkylthio in which the aryl may be substituted with C₁₋₄ alkyl, hydroxy or halogen; or R² represents an imidazolylthio group in which the imidazolyl moiety may be substituted with one or more substituents selected from C₁₋₄ alkyl and C-substituted with nitro; or R² represents an amino group which is mono- or di-substituted by one or two substituents selected from C₁₋₆ alkyl, C₁₋₆ alkoxy, C₁₋₆ hydroxyalkyl, C₃₋₇ cycloalkyl optionally substituted by C₁₋₆ alkyl, aryl, aralkyl in which the aryl may optionally be substituted with C₁₋₄ alkyl, hydroxy or halogen, allyl optionally substituted with mono- or di-alkyl or alkoxy groups; and R³ represents hydrogen, amino or C₁₋₆ alkyl; or a pharmaceutically acceptable derivative thereof which comprises preparing said compound by a process involving:
A) treating a compound of formula (II) 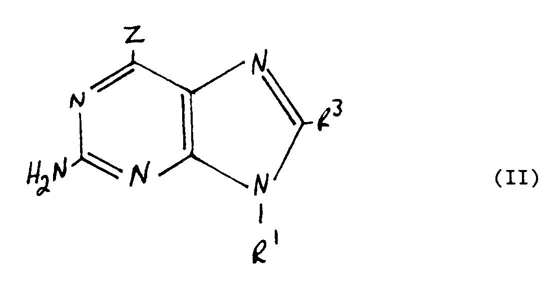 wherein R¹ and R³ are as hereinbefore defined and Z represents a precursor group for the said R² group with an agent or under conditions serving to convert the precursor Z group to the desired R² group; or Z represents a thio group onto which may be substituted an appropriate group to form a compound of formula (I) wherein R² is a substituted thio group; or
B) reacting a compound of formula (III) 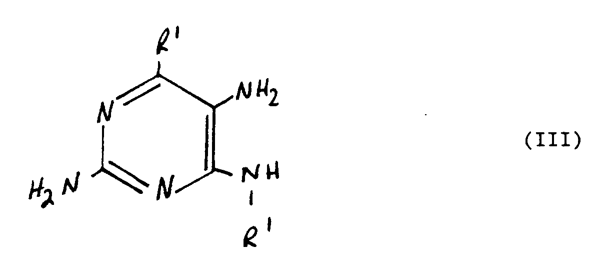 (wherein R¹ and R² are hereinbefore defined) or a pharmaceutically acceptable derivative thereof, with an agent serving to effect formation of the imidazole ring in the desired compound of formula (I); or
i) where a compound of formula (I) is formed, converting the said compound to a pharmaceutically acceptable derivative thereof; or
ii) where a pharmaceutically acceptable derivative of a compound of formula (I) is formed, converting the said derivative to the parent compound of formula (I) or to a further such derivative.
and bringing the resulting compound of formula (I) into association with at least one pharmaceutical excipient to form the said pharmaceutical formulation.

6. A process according to Claim 1 for the preparation of a pharmaceutical formulation comprising an active ingredient (±)-cis-4-[2-amino-(6-cyclopropylamino)-9H-purin-9-yl]-2-cyclopentene-1-methanol.

## Patentansprüche (Patentansprüche für folgende(n) Vertragsstaat(en): AT, BE, CH, DE, FR, GB, IT, LI, LU, NL, SE)

1. Verbindung der Formel (I): 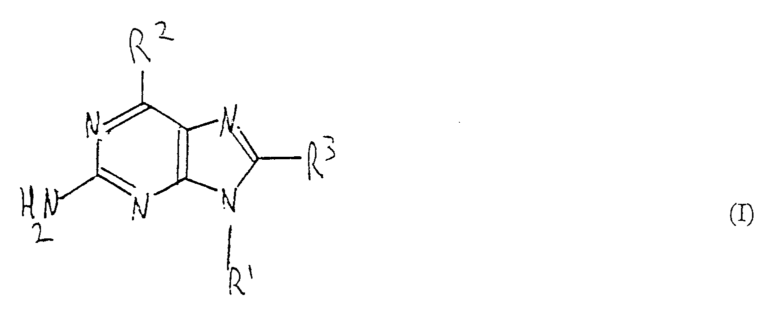 worin bedeuten:
R¹ 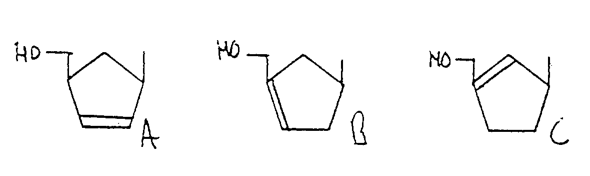 R² verzweigtes oder geradkettiges C₁₋₆-Alkoxy, wahlweise substituiert durch C₁₋₆-Alkoxy, C₃₋₆-Cycloalkyl; C₃₋₆-Cycloalkyl; C₃₋₈-Cycloalkyloxy; Aryloxy; Aralkyl oder Aralkyloxy, worin das Aryl wahlweise substituiert sein kann mit C₁₋₄-Alkyl, Hydroxy oder Halogen; C₂-C₆ Alkenylthio; C₃₋₆-Cycloalkylthio; C₁₋₆-Alkylthio; Arylthio oder Aralkylthio, worin das Aryl wahlweise substituiert sein kann mit C₁₋₄-Alkyl, Hydroxy, Halogen oder Nitro; oder R² bedeutet eine heterocyclische Gruppe, enthaltend ein Sauerstoffatom oder ein oder zwei Stickstoffatome und 3-7 Kohlenstoffatome mit wahlweise Doppelbindungen im Ring und enthaltend wahlweise ein oder mehrere Heteroatome, gewählt aus Schwefel und Sauerstoff sowie wahlweise am Ring substituiert durch eine oder mehrere C₁₋₄-Alkyl-, Hydroxy- oder Halogengruppen, C₃₋₆-Cycloalkylthio, Aralkylthio, worin das Aryl mit C₁₋₄-Alkyl, Hydroxy oder Halogen substituiert sein kann; oder R² bedeutet eine Imidazolylthiogruppe, worin der Imidazolylteil mit einem oder mehreren Substituenten, gewählt aus C₁₋₄-Alkyl und C-substituiert mit Nitro, substituiert sein kann: oder R² bedeutet eine Aminogruppe, welche mono- oder disubstituiert ist durch einen oder zwei Substituenten, gewählt aus C₁₋₆-Alkyl, C₁₋₆-Alkoxy, C₁₋₆-Hydroxyalkyl, C₃₋₇-Cycloalkyl, wahlweise substituiert durch C₁₋₆-Alkyl, Aryl, Aralkyl, worin das Aryl wahlweise substituiert sein kann mit C₁₋₄-Alkyl, Hydroxy oder Halogen, Allyl, wahlweise substituiert mit Mono- oder Dialkyl- oder Alkoxygruppen; und
R³ Wasserstoff, Amino oder C₁₋₆-Alkyl;
oder ein pharmazeutisch annehmbares Derivat davon.

2. Verbindung der Formel (I) nach Anspruch 1, worin R¹ die Bedeutung A hat

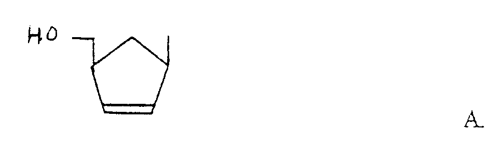

3. Verbindung nach Anspruch 1 der Formel: 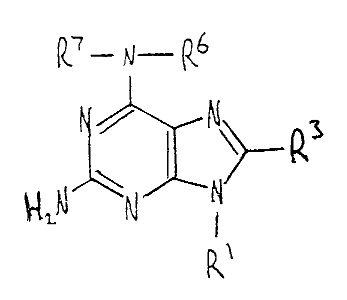 worin bedeuten:
R¹ 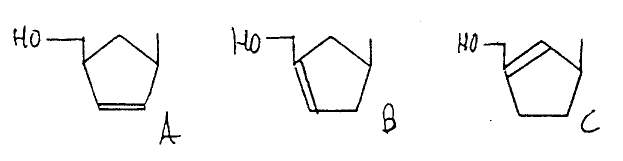 R³ Wasserstoff, Amino der C₁₋₆-Alkyl;
R⁶ C₃₋₆-Cycloalkyl; und
R⁷ ein Wasserstoffatom der ein Substituent, gewählt aus C₁₋₆-Alkyl, C₁₋₆-Alkoxy, C₁₋₆-Hydroxyalkyl, C₃₋₆-Cycloalkyl, wahlweise substituiert durch C₁₋₆-Alkyl, Aryl, Aralkyl, worin das Aryl wahlweise substituiert sein kann mit C₁₋₄Alkyl, Hydroxy oder Halogen, Allyl, wahlweise substituiert mit Mono- oder Dialkyl- oder Alkoxygruppen;
oder ein pharmazeutisch annehmbares Derivat davon.

4. (±)-cis-4-[2-Amino-(6-cyclopropylamino)-9H-purin-9-yl]-2-cyclopenten-1-methanol.

5. Pharmazeutisch annehmbares Derivat der Verbindung (±)-cis-4-[2-Amino-(6-cyclopropylamino)-9H-purin-9-yl]-2-cyclopenten-1-methanol.

6. Pharmazeutisch annehmbares Salz der Verbindung (±)-cis-4-[2-Amino-(6-cyclopropylamino)-9H-purin-9-yl]-2-cyclopenten-1-methanol.

7. Verbindung nach mindestens einem der Ansprüche 1 bis 5 oder ein pharmazeutisch annehmbares Derivat davon zur Verwendung in der medizinischen Therapie.

8. Verbindung nach mindestens einem der Ansprüche 1 bis 5 zur Verwendung bei der Behandlung oder Prophylaxe von HIV-Infektionen.

9. Verbindung nach mindestens einem der Ansprüche 1 bis 5 zur Verwendung bei der Behandlung oder Prophylaxe von HBV-Infektionen.

10. Verbindung nach mindestens einem der Ansprüche 1 bis 5 zur Verwendung bei der Herstellung eines Arzneimittels zur Behandlung oder Prophylaxe von Retrovirusinfektionen oder HBV-Infektionen.

11. Verfahren zur Herstellung einer Verbindung der Formel (I) wie in Anspruch 1 definiert, umfassend:
A) Behandeln einer Verbindung der Formel (II) 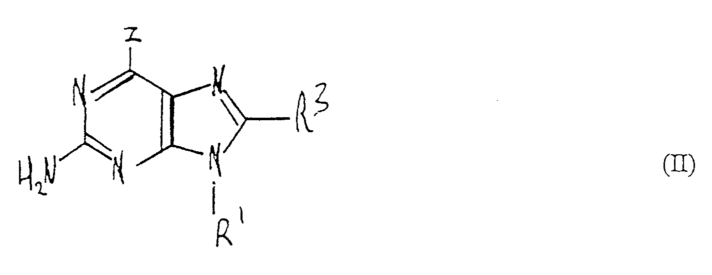 worin bedeuten:
R¹ 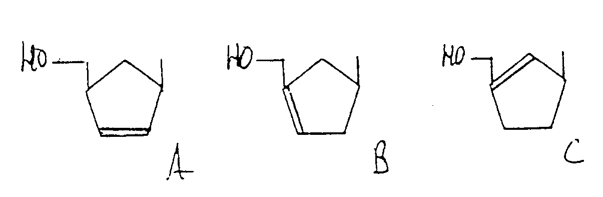 worin R¹ und R³ wie oben definiert sind und Z eine Vorläufergruppe für die R²-Gruppe bedeutet, mit einem Mittel oder unter Bedingungen, welche dazu dienen, die Z-Vorläufergruppe in die erwünschte R²-Gruppe umzuwandeln; oder Z bedeutet eine Thiogruppe, auf welcher eine geeignete Gruppe substituiert sein kann, um eine Verbindung der Formel (I) zu bilden, worin R² eine substituierte Thiogruppe ist, oder
B) Umsetzen einer Verbindung der Formel (III) 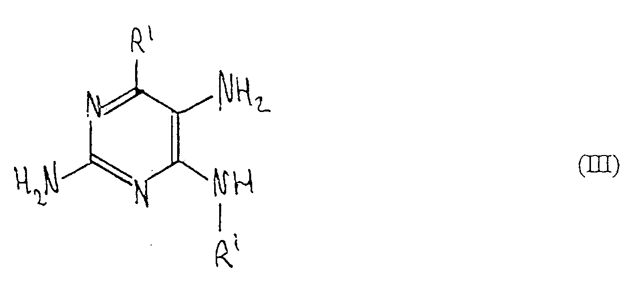 (worin R¹ und R² wie oben definiert sind) oder eines pharmazeutisch annehmbaren Derivats davon, mit einem Mittel, welches dazu dient, die Bildung des Imidazolrings in der erwünschten Verbindung der Formel (I) zu bewirken; oder
i) wenn eine Verbindung der Formel (I) gebildet wird, Umwandeln der Verbindung in ein pharmazeutisch annehmbares Derivat davon; oder
ii) wenn ein pharmazeutisch annehmbares Derivat einer Verbindung der Formel (I) gebildet wird, Umwandeln des Derivats in die Stammverbindung der Formel (I) oder in ein weiteres Derivat.

12. Pharmazeutische Zubereitung, umfassend als Wirkstoff eine Verbindung der Formel (I) wie in mindestens einem der Ansprüche 1 bis 5 definiert, und einen pharmazeutisch annehmbaren Träger hierfür.

13. Pharmazeutische Zubereitung nach Anspruch 11, wobei die pharmazeutische Zubereitung zu einer Tablette oder einer Kapsel ausgebildet ist.

## Patentansprüche (Patentansprüche für folgende(n) Vertragsstaat(en): ES, GR)

1. Verfahren zur Herstellung einer Verbindung der Formel (I) 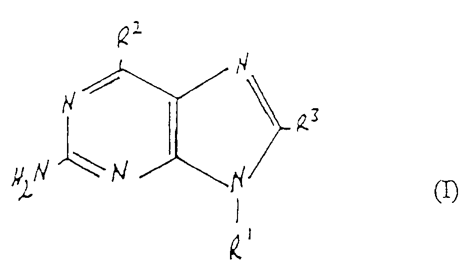 worin bedeuten:
R¹ 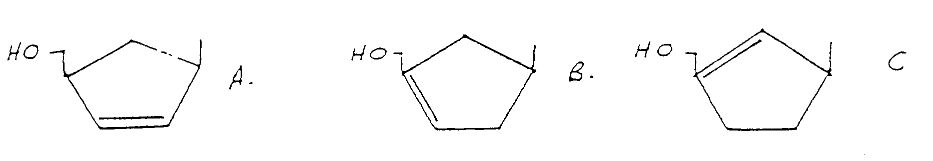 R² verzweigtes oder geradkettiges C₁₋₆-Alkoxy, wahlweise substituiert durch C₁₋₆-Alkoxy, C₃₋₆-Cycloalkyl; C₃₋₆-Cycloalkyl; C₃₋₈-Cycloalkyloxy; Aryloxy; Aralkyl oder Aralkyloxy, worin das Aryl wahlweise substituiert sein kann mit C₁₋₄-Alkyl, Hydroxy oder Halogen; C₁₋₆-Alkenylthio; C₃₋₆-Cycloalkylthio; C₁₋₆-Alkylthio; Arylthio oder Aralkylthio, worin das Aryl wahlweise substituiert sein kann mit C₁₋₄-Alkyl, Hydroxy, Halogen oder Nitro; oder R² bedeutet eine heterocyclische Gruppe, enthaltend ein Sauerstoffatom oder ein oder zwei Stickstoffatome und 3-7 Kohlenstoffatome mit wahlweise Doppelbindungen im Ring und enthaltend wahlweise ein oder mehrere Heteroatome, gewählt aus Schwefel und Sauerstoff sowie wahlweise am Ring substituiert durch eine oder mehrere C₁₋₄-Alkyl-, Hydroxy- oder Halogengruppen. C₃₋₆-Cycloalkylthio, Aralkylthio, worin das Aryl mit C₁₋₄-Alkyl, Hydroxy oder Halogen substituiert sein kann; oder R² bedeutet eine Imidazolylthiogruppe, worin der Imidazolylteil mit einem oder mehreren Substituenten, gewählt aus C₁₋₄-Alkyl und C-substituiert mit Nitro, substituiert sein kann; oder R² bedeutet eine Aminogruppe, welche mono- oder disubstituiert ist durch einen oder zwei Substituenten, gewählt aus C₁₋₆-Alkyl, C₁₋₆-Alkoxy, C₁₋₆-Hydroxyalkyl, C₃₋₇-Cycloalkyl, wahlweise substituiert durch C₁₋₆-Alkyl, Aryl, Aralkyl, worin das Aryl wahlweise substituiert sein kann mit C₁₋₄-Alkyl, Hydroxy oder Halogen, Allyl, wahlweise substituiert mit Mono- oder Dialkyl- oder Alkoxygruppen; und
R³ Wasserstoff, Amino oder C₁₋₆-Alkyl;
oder eines pharmazeutisch annehmbaren Derivats davon,
umfassend:
A) Behandeln einer Verbindung der Formel (II) 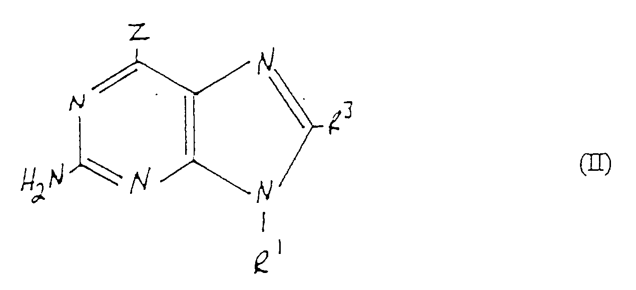 worin R¹ und R³ wie oben definiert sind und Z eine Vorläufergruppe für die R²-Gruppe bedeutet, mit einem Mittel oder unter Bedingungen, welche dazu dienen, die Z-Vorläufergruppe in die erwünschte R²-Gruppe umzuwandeln; oder Z bedeutet eine Thiogruppe, auf welcher eine geeignete Gruppe substituiert sein kann, um eine Verbindung der Formel (I) zu bilden, worin R² eine substituierte Thiogruppe ist, oder
B) Umsetzen einer Verbindung der Formel (III) 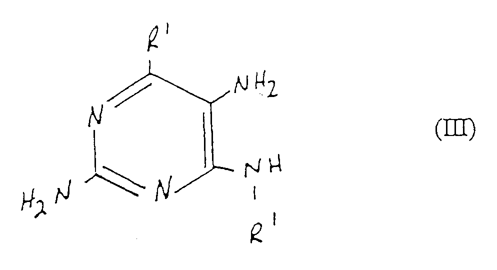 (worin R¹ und R² wie oben definiert sind) oder eines pharmazeutisch annehmbaren Derivats davon, mit einem Mittel, welches dazu dient, die Bildung des Imidazolrings in der erwünschten Verbindung der Formel (I) zu bewirken; oder
i) wenn eine Verbindung der Formel (I) gebildet wird, Umwandeln der Verbindung in ein pharmazeutisch annehmbares Derivat davon; oder
ii) wenn ein pharmazeutisch annehmbares Derivat einer Verbindung der Formel (I) gebildet wird, Umwandeln des Derivats in die Stammverbindung der Formel (I) oder in ein weiteres Derivat.

2. Verfahren nach Anspruch 1 zur Herstellung einer Verbindung der Formel (I), worin R¹ die Bedeutung A hat

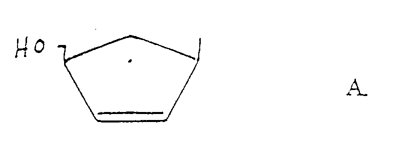

3. Verfahren nach den Ansprüchen 1 oder 2 zur Herstellung einer Verbindung der Formel: 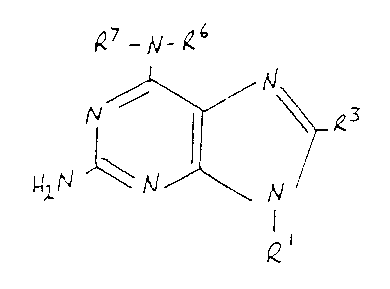 worin bedeuten:
R¹ 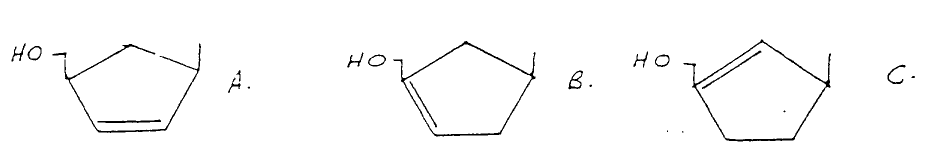 R³ Wasserstoff, Amino der C₁₋₆-Alkyl;
R⁶ C₃₋₆-Cycloalkyl; und
R⁷ ein Wasserstoffatom oder ein Substituent, gewählt aus C₁₋₆-Alkyl, C₁₋₆-Alkoxy, C₁₋₆-Hydroxyalkyl, C₃₋₆-Cycloalkyl, wahlweise substituiert durch C₁₋₆-Alkyl, Aryl, Aralkyl, worin das Aryl wahlweise substituiert sein kann mit C₁₋₄-Alkyl, Hydroxy oder Halogen, Allyl, wahlweise substituiert mit Mono- oder Dialkyl- oder Alkoxygruppen;
oder eines pharmazeutisch annehmbaren Derivats davon.

4. Verfahren nach Anspruch 1 zur Herstellung von (±)-cis-4-[2-Amino-(6-cyclopropylamino)-9H-purin-9-yl]-2-cyclopenten-1-methanol.

5. Verfahren zur Herstellung einer pharmazeutischen Zubereitung, enthaltend eine Verbindung der Formel (I) 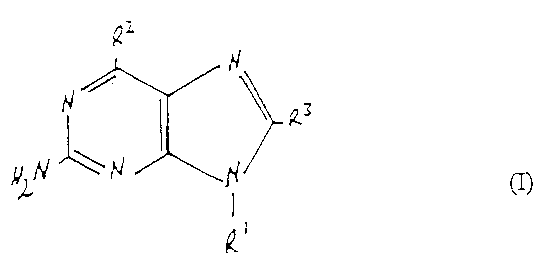 worin bedeuten:
R¹ 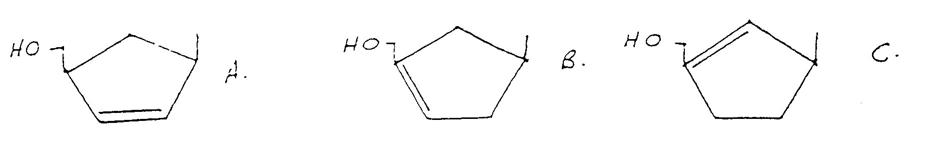 R² verzweigtes oder geradkettiges C₁₋₆-Alkoxy, wahlweise substituiert durch C₁₋₆-Alkoxy, C₃₋₆-Cycloalkyl; C₃₋₆-Cycloalkyl; C₃₋₈-Cycloalkyloxy; Aryloxy; Aralkyl oder Aralkyloxy, worin das Aryl wahlweise substituiert sein kann mit C₁₋₄-Alkyl, Hydroxy oder Halogen; C₁₋₆-Alkenylthio; C₃₋₆-Cycloalkylthio; C₁₋₆-Alkylthio; Arylthio oder Aralkylthio, worin das Aryl wahlweise substituiert sein kann mit C₁₋₄-Alkyl, Hydroxy, Halogen oder Nitro; oder R² bedeutet eine heterocyclische Gruppe enthaltend ein Sauerstoffatom oder ein oder zwei Stickstoffatome und 3-7 Kohlenstoffatome mit wahlweise Doppelbindungen im Ring und enthaltend wahlweise ein oder mehrere Heteroatome, gewählt aus Schwefel und Sauerstoff sowie wahlweise am Ring substituiert durch eine oder mehrere C₁₋₄-Alkyl-, Hydroxy- oder Halogengruppen, C₃₋₆-Cycloalkylthio, Aralkylthio, worin das Aryl mit C₁₋₄-Alkyl, Hydroxy oder Halogen substituiert sein kann; oder R² bedeutet eine Imidazolylthiogruppe, worin der Imidazolylteil mit einem oder mehreren Substituenten, gewählt aus C₁₋₄-Alkyl und C-substituiert mit Nitro, substituiert sein kann; oder R² bedeutet eine Aminogruppe, welche mono- oder disubstituiert ist durch einen oder zwei Substituenten, gewählt aus C₁₋₆-Alkyl, C₁₋₆-Alkoxy, C₁₋₆-Hydroxyalkyl, C₃₋₇-Cycloalkyl, wahlweise substituiert durch C₁₋₆-Alkyl, Aryl, Aralkyl, worin das Aryl wahlweise substituiert sein kann mit C₁₋₄-Alkyl, Hydroxy oder Halogen, Allyl, wahlweise substituiert mit Mono- oder Dialkyl- oder Alkoxygruppen; und
R³ Wasserstoff, Amino oder C₁₋₆-Alkyl;
oder ein pharmazeutisch annehmbares Derivat davon,
umfassend die Herstellung der Verbindung durch ein Verfahren, beinhaltend:
A) Behandeln einer Verbindung der Formel (II) 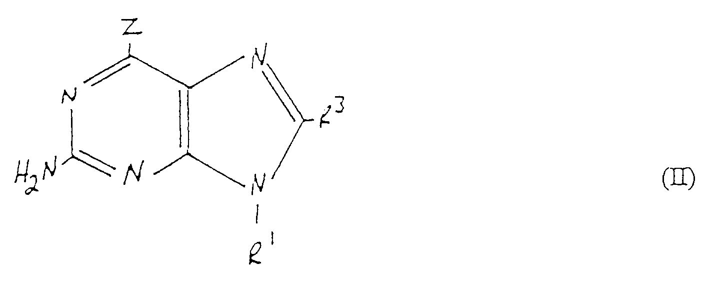 worin R¹ und R³ wie oben definiert sind und Z eine Vorläufergruppe für die R²-Gruppe bedeutet, mit einem Mittel oder unter Bedingungen, welche dazu dienen, die Z-Vorläufergruppe in die erwünschte R²-Gruppe umzuwandeln; oder Z bedeutet eine Thiogruppe, auf welcher eine geeignete Gruppe substituiert sein kann, um eine Verbindung der Formel (I) zu bilden, worin R² eine substituierte Thiogruppe ist, oder
B) Umsetzen einer Verbindung der Formel (III) 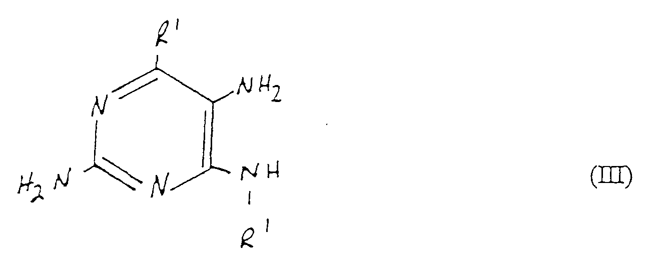 (worin R¹ und R² wie oben definiert sind) oder eines pharmazeutisch annehmbaren Derivats davon, mit einem Mittel, welches dazu dient, die Bildung des Imidazolrings in der erwünschten Verbindung der Formel (I) zu bewirken; oder
i) wenn eine Verbindung der Formel (I) gebildet wird, Umwandeln der Verbindung in ein pharmazeutisch annehmbares Derivat davon; oder
ii) wenn ein pharmazeutisch annehmbares Derivat einer Verbindung der Formel (I) gebildet wird, Umwandeln des Derivats in die Stammverbindung der Formel (I) oder in ein weiteres Derivat,
und Zusammenbringen der resultierenden Verbindung der Formel (I) mit mindestens einem pharmazeutischen Träger zur Bildung der pharmazeutischen Zubereitung.

6. Verfahren nach Anspruch 1, zur Herstellung einer pharmazeutischen Zubereitung, umfassend als Wirkstoff (±)-cis-4-[2-Amino-(6-cyclopropylamino)-9H-purin-9-yl]-2-cyclopenten-1-methanol.

## Revendications (Revendications pour l'(les) Etat(s) contractant(s) suivant(s): AT, BE, CH, DE, FR, GB, IT, LI, LU, NL, SE)

1. Composé de formule (I) : 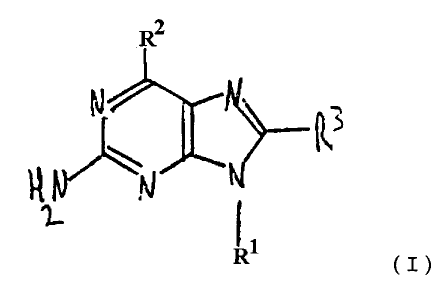 dans laquelle R¹ représente 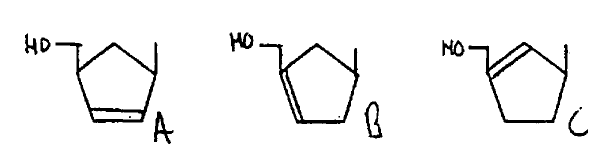 et R² représente une chaîne alkoxy en C₁₋₆ droite ou ramifiée, éventuellement substituée par alkoxy en C₁₋₆, cycloalkyle en C₃₋₆ ; un cycloalkyle en C₃₋₆ ; un cycloalkyloxy en C₃₋₈ ; un aryloxy ; un aralkyle ou un aralkyloxy dont l'aryle peut éventuellement être substitué par alkyle en C₁₋₄, hydroxy ou halogène ; en C₂-C₆ un alkénylthio en C₁₋₆ ; un cycloalkylthio en C₃₋₆ ; un alkylthio en C₁₋₆ ; un arylthio ou un aralkylthio dont l'aryle peut éventuellement être substitué par alkyle en C₁₋₄, hydroxy, halogène ou nitro ; ou bien R² représente un groupe hétérocyclique contenant un atome d'oxygène ou un ou deux atomes d'azote, et 3 à 7 atomes de carbone avec éventuellement des doubles liaisons dans le cycle et contenant éventuellement un ou plusieurs hétéroatomes choisis parmi le soufre et l'oxygène et éventuellement substitué dans le cycle par un ou plusieurs groupements alkyle en C₁₋₄, hydroxy ou halogène, cycloalkylthio en C₃₋₆, aralkylthio dans lequel l'aryle peut être substitué par alkyle en C₁₋₄, hydroxy ou halogène ; ou bien R² représente un groupement imidazolylthio dans lequel la partie imidazolyle peut être substituée par un ou plusieurs substituants choisis parmi alkyle en C₁₋₄ et nitro porté par un carbone ; ou bien R² représente un groupement amino qui est mono- ou bi-substitué par un ou deux substituants choisis parmi alkyle en C₁₋₆, alkoxy en C₁₋₆, hydroxyalkyle en C₁₋₆, cycloalkyle en C₃₋₇ éventuellement substitué par alkyle en C₁₋₆, aryle, aralkyle dont l'aryle peut éventuellement être substitué par alkyle en C₁₋₄, hydroxy ou halogène, allyle éventuellement substitué par des groupements mono- ou di-alkyle ou alkoxy ; et R³ représente un hydrogène, un groupement amino ou un alkyle en C₁₋₆ ; ou un dérivé pharmaceutiquement acceptable de celui-ci.

2. Composé de formule (I) selon la revendication 1, dans laquelle R¹ représente A.

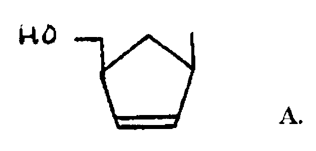

3. Composé selon la revendication 1, de formule : 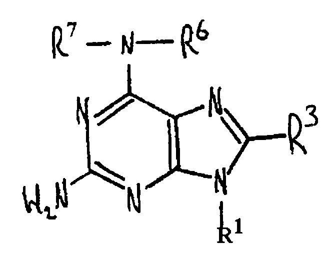 dans laquelle R¹ représente 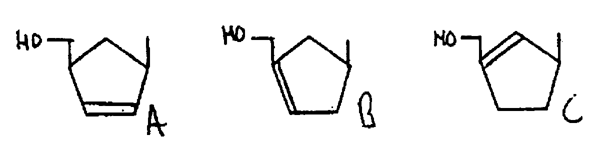 R³ représente un hydrogène, un groupement amino ou un alkyle en C₁₋₆ ; R⁶ représente un cycloalkyle en C₃₋₆ ; et R⁷ représente un atome d'hydrogène ou un substituant choisi parmi un alkyle en C₁₋₆, un alkoxy en C₁₋₆, un hydroxyalkyle en C₁₋₆, un cycloalkyle en C₃₋₆ éventuellement substitué par alkyle en C₁₋₆, aryle, aralkyle dont l'aryle peut éventuellement être substitué par alkyle en C₁₋₄, hydroxy ou halogène, allyle éventuellement substitué par des groupements mono- ou di-alkyle ou alkoxy ; ou un dérivé pharmaceutiquement acceptable de celui-ci.

4. (±)-cis-4-[2-amino-(6-cyclopropylamino)-9H-purine-9-yl]-2-cyclopentène-1-méthanol.

5. Dérivé pharmaceutiquement acceptable du composé (±)-cis-4-[2-amino-(6-cyclopropylamino)-9H-purine-9-yl]-2-cyclopentène-1-méthanol.

6. Sel pharmaceutiquement acceptable du composé (±)-cis-4-[2-amino-(6-cyclopropylamino)-9H-purine-9-yl]-2-cyclopentène-1-méthanol.

7. Composé selon l'une quelconque des revendications 1 à 5 ou dérivé pharmaceutiquement acceptable de celui-ci pour utilisation en thérapie médicale.

8. Composé selon l'une quelconque des revendications 1 à 5 pour utilisation dans le traitement curatif ou préventif des infections par VIH.

9. Composé selon l'une quelconque des revendications 1 à 5 pour utilisation dans le traitement curatif ou préventif des infections par virus de l'hépatite B.

10. Composé selon l'une quelconque des revendications 1 à 5 pour utilisation dans le traitement curatif ou préventif des infections par des rétrovirus ou par virus de l'hépatite B.

11. Procédé de préparation d'un composé de formule (I) tel que défini dans la revendication 1 comprenant les étapes consistant à :
A) traiter un composé de formule (II) 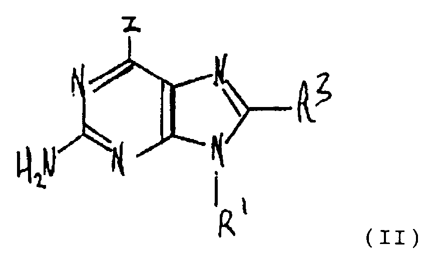 dans laquelle R¹ représente 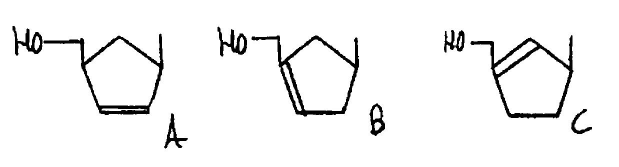 dans laquelle R¹ et R³ sont tels que définis ci-dessus et Z représente un groupement précurseur dudit groupement R² avec un agent ou dans des conditions servant à convertir le groupement précurseur Z en le groupe R² désiré ; ou Z représente un groupement thio pouvant être substitué par un groupement approprié pour former un composé de formule (I) dans laquelle R² est un groupement thio substitué ; ou
B) faire réagir un composé de formule (III) 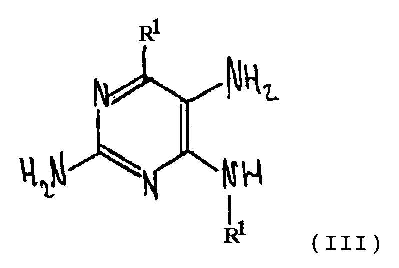 (dans laquelle R¹ et R² sont tels que définis ci-dessus) ou un dérivé pharmaceutiquement acceptable de celui-ci, avec un agent servant à effectuer la formation du cycle imidazole dans le composé désiré de formule (I) ; ou
i) lorsqu'un composé de formule (I) est formé, convertir ledit composé en un dérivé pharmaceutiquement acceptable de celui-ci ; ou
ii) lorsqu'un dérivé pharmaceutiquement acceptable du composé de formule (I) est formé, convertir ledit dérivé en ledit composé de formule (I) ou en un autre dérivé de celui-ci.

12. Formulation pharmaceutique comprenant comme ingrédient actif un composé de formule (I) tel que défini dans l'une quelconque des revendications 1 à 5 et un support pharmaceutiquement acceptable.

13. Formulation pharmaceutique selon la revendication 11 dans laquelle la formulation pharmaceutique est présentée sous forme d'un comprimé ou d'une capsule.

## Revendications (Revendications pour l'(les) Etat(s) contractant(s) suivant(s): ES, GR)

1. Procédé de synthèse d'un composé de formule I: 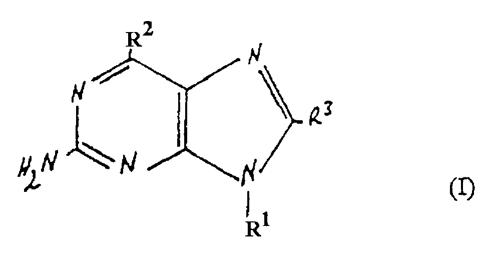 dans laquelle R¹ représente 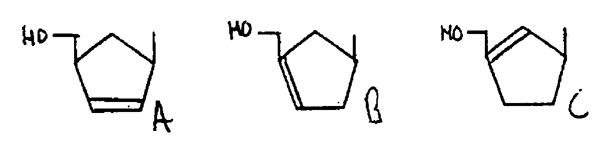 et R² représente une chaîne alkoxy en C₁₋₆ droite ou ramifiée, éventuellement substituée par alkoxy en C₁₋₆, cycloalkyle en C₃₋₆ ; cycloalkyle en C₃₋₆ ; cycloalkyloxy en C₃₋₈ ; aryloxy ; aralkyle ou aralkyloxy dont l'aryle peut éventuellement être substitué par alkyle en C₁₋₄, hydroxy ou halogène ; alkénylthio en C₁₋₆ ; cycloalkylthio en C₃₋₆ ; alkylthio en C₁₋₆ ; arylthio ou aralkylthio dont l'aryle peut éventuellement être substitué par alkyle en C₁₋₄, hydroxy, halogène ou nitro ; ou bien R² représente un groupe hétérocyclique contenant un atome d'oxygène ou un ou deux atomes d'azote, et 3 à 7 atomes de carbone avec éventuellement des doubles liaisons dans le cycle et contenant éventuellement un ou plusieurs hétéroatomes choisis parmi le soufre et l'oxygène et éventuellement substitué dans le cycle par un ou plusieurs groupements alkyle en C₁₋₄, hydroxy ou halogène, cycloalkylthio en C₃₋₆, aralkylthio dans lequel l'aryle peut être substitué par alkyle en C₁₋₄, hydroxy ou halogène ; ou bien R² représente un groupement imidazolylthio dans lequel la partie imidazolyle peut être substituée par un ou plusieurs substituants choisis parmi alkyle en C₁₋₄ et nitro porté par un carbone ; ou bien R² représente un groupement amino qui est mono- ou bi-substitué par un ou deux substituants choisis parmi alkyle en C₁₋₆, alkoxy en C₁₋₆, hydroxyalkyle en C₁₋₆, cycloalkyle en C₃₋₇ éventuellement substitué par alkyle en C₁₋₆, aryle, aralkyle dont l'aryle peut éventuellement être substitué par alkyle en C₁₋₄, hydroxy ou halogène, allyle éventuellement substitué par des groupements mono- ou di-alkyle ou alkoxy ; et R³ représente un hydrogène, un groupement amino ou un alkyle en C₁₋₆ ; ou un dérivé pharmaceutiquement acceptable dudit composé.
comprenant les étapes consistant à :
A) traiter un composé de formule (II) 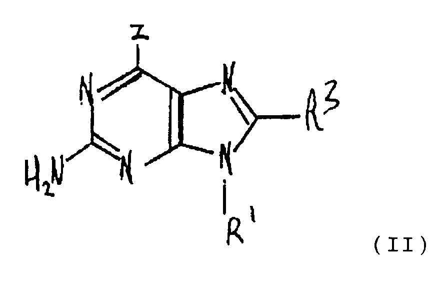 dans laquelle R¹ et R³ sont tels que définis ci-dessus et Z représente un groupement précurseur dudit groupement R² avec un agent ou dans des conditions servant à convertir le groupement précurseur Z en le groupe R² désiré ; ou Z représente un groupement thio pouvant être substitué par un groupement approprié pour former un composé de formule (I) dans laquelle R² est par un groupement thio substitué ; ou
B) faire réagir un composé de formule (III) 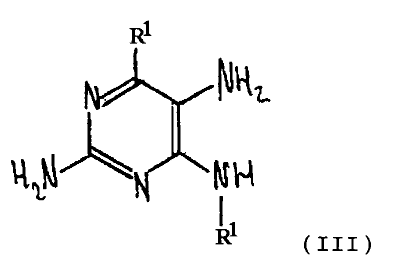 (dans laquelle R¹ et R² sont tels que définis ci-dessus) ou un dérivé pharmaceutiquement acceptable de celui-ci, avec un agent servant à effectuer la formation du cycle imidazole dans le composé désiré de formule (I) ; ou
i) lorsqu'un composé de formule (I) est formé, convertir ledit composé en un dérivé pharmaceutiquement acceptable de celui-ci ; ou
ii) lorsqu'un dérivé pharmaceutiquement acceptable du composé de formule (I) est formé, convertir ledit dérivé en ledit composé de formule (I) ou en un autre dérivé de celui-ci.

2. Procédé selon la revendication 1 pour la préparation d'un composé de formule (I) dans laquelle R¹ représente

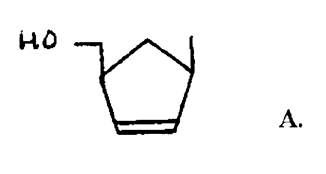

3. Procédé selon la revendication 1 ou 2 pour la préparation d'un composé de formule : 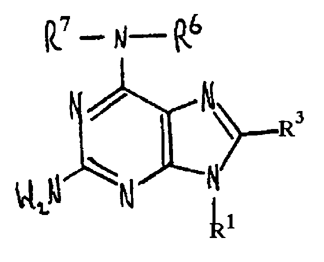 dans laquelle R¹ représente 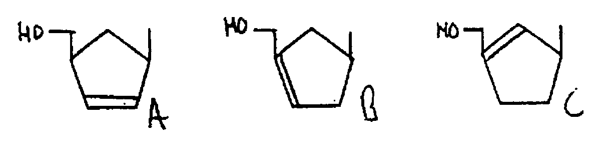 R³ représente un hydrogène, un groupement amino ou un alkyle en C₁₋₆ ; R⁶ représente un cycloalkyle en C₃₋₆ ; et R⁷ représente un atome d'hydrogène ou un substituant choisi parmi alkyle en C₁₋₆, alkoxy en C₁₋₆, hydroxyalkyle en C₁₋₆, cycloalkyle en C₃₋₆ éventuellement substitué par alkyle en C₁₋₆, aryle, aralkyle dont l'aryle peut éventuellement être substitué par alkyle en C₁₋₄, hydroxy ou halogène, allyle éventuellement substitué par des groupements mono- ou di-alkyle ou alkoxy ; ou d'un dérivé pharmaceutiquement acceptable de ce composé.

4. Procédé selon la revendication 1 pour la prépration du (±)-cis-4-[2-amino-(6-cyclopropylamino)-9H-purine-9-yl]-2-cyclopentène-1-méthanol.

5. Procédé de préparation d'une formulation pharmaceutique contenant un composé de formule I. 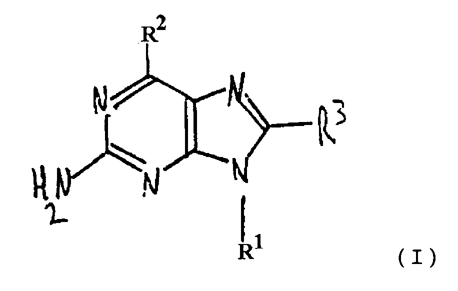 dans laquelle R¹ représente 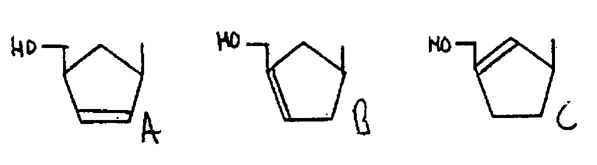 et R² représente une chaîne alkoxy en C₁₋₆ droite ou ramifiée, éventuellement substituée par alkoxy en C₁₋₆, cycloalkyle en C₃₋₆ ; un cycloalkyle en C₃₋₆ ; un cycloalkyloxy en C₃₋₈ ; un aryloxy ; un aralkyle ou un aralkyloxy dont l'aryle peut éventuellement être substitué par alkyle en C₁₋₄, hydroxy ou halogène ; un alkénylthio en C₁₋₆ ; un cycloalkylthio en C₃₋₆ ; un alkylthio en C₁₋₆ ; un arylthio ou un aralkylthio dont l'aryle peut éventuellement être substitué par alkyle en C₁₋₄, hydroxy, halogène ou nitro ; ou bien R² représente un groupe hétérocyclique contenant un atome d'oxygène ou un ou deux atomes d'azote, et 3 à 7 atomes de carbone avec éventuellement des doubles liaisons dans le cycle et contenant éventuellement un ou plusieurs hétéroatomes choisis parmi le soufre et l'oxygène et éventuellement substitué dans le cycle par un ou plusieurs groupements alkyle en C₁₋₄, hydroxy ou halogène, cycloalkylthio en C₃₋₆, aralkylthio dans lequel l'aryle peut être substitué par alkyle en C₁₋₄, hydroxy ou halogène ; ou bien R² représente un groupement imidazolylthio dans lequel la partie imidazolyle peut être substituée par un ou plusieurs substituants choisis parmi alkyle en C₁₋₄ et nitro porté par un carbone ; ou bien R² représente un groupement amino qui est mono- ou bi-substitué par un ou deux substituants choisis parmi alkyle en C₁₋₆, alkoxy en C₁₋₆, hydroxyalkyle en C₁₋₆, cycloalkyle en C₃₋₇ éventuellement substitué par alkyle en C₁₋₆, aryle, aralkyle dont l'aryle peut éventuellement être substitué par alkyle en C₁₋₄, hydroxy ou halogène, allyle éventuellement substitué par des groupements mono- ou di-alkyle ou alkoxy ; et R³ représente un hydrogène, un groupement amino ou un alkyle en C₁₋₆ ; ou un dérivé pharmaceutiquement acceptable de celui-ci, qui comprend la préparation dudit composé par un procédé comprenant les étapes consistant à :
A) traiter un composé de formule (II) 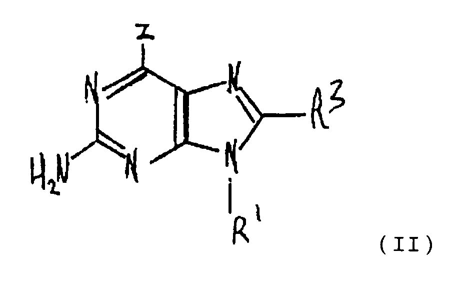 dans laquelle R¹ et R³ sont tels que définis ci-dessus et Z représente un groupement précurseur dudit groupement R² avec un agent ou dans des conditions servant à convertir le groupement précurseur Z en le groupe R² désiré ; ou Z représente un groupement thio pouvant être substitué par un groupement approprié pour former un composé de formule (I) dans laquelle R² est un groupement thio substitué ; ou
B) faire réagir un composé de formule (III) 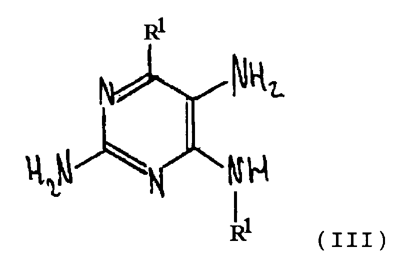 (dans laquelle R¹ et R² sont tels que définis ci-dessus) ou un dérivé pharmaceutiquement acceptable de celui-ci, avec un agent servant à effectuer la formation du cycle imidazole dans le composé désiré de formule (I) ; ou
i) lorsqu'un composé de formule (I) est formé, convertir ledit composé en un dérivé pharmaceutiquement acceptable de celui-ci ; ou
ii) lorsqu'un dérivé pharmaceutiquement acceptable de formule (I) est formé, convertir ledit dérivé en ledit composé de formule (I) ou en un autre dérivé de celui-ci, et mettre le composé de formule I en association avec au moins un excipient pharmaceutique pour former ladite formulation pharmaceutique.

6. Procédé selon la revendication 1 pour la préparation d'une formulation pharmaceutique comprenant un ingrédient actif (±)-cis-4-[2-amino-(6-cyclopropylamino)-9H-purine-9-yl]-2-cyclopentène-1-méthanol.
